(19) Europäisches Patentamt
European Patent Office
Office européen des brevets

(11) EP 0 752 846 B1

(12) **EUROPEAN PATENT SPECIFICATION**

(45) Date of publication and mention of the grant of the patent:
**01.08.2001 Bulletin 2001/31**

(21) Application number: **95912678.0**

(22) Date of filing: **01.03.1995**

(51) Int Cl.$^7$: **A61K 7/50**

(86) International application number:
**PCT/US95/02588**

(87) International publication number:
**WO 95/26710 (12.10.1995 Gazette 1995/43)**

(54) **SKIN MOISTURIZING AND CLEANSING BAR COMPOSITION**

HAUTFEUCHTHALTENDE UND REINIGENDE ZUSAMMENSETZUNG IN STUECKFORM

COMPOSITION D'HYDRATATION ET DE NETTOYAGE POUR LA PEAU SOUS FORME DE PAIN

(84) Designated Contracting States:
**AT BE CH DE DK ES FR GB GR IE IT LI LU NL PT SE**

(30) Priority: **30.03.1994 US 220354**

(43) Date of publication of application:
**15.01.1997 Bulletin 1997/03**

(73) Proprietor: **THE PROCTER & GAMBLE COMPANY**
**Cincinnati, Ohio 45202 (US)**

(72) Inventors:
- **KACHER, Mark, Leslie**
  **Mason, OH 45040 (US)**
- **GEARY, Nicholas, William**
  **Cincinnati, OH 45242 (US)**
- **EVANS, Marcus, Wayne**
  **Cincinnati, OH 45231 (US)**
- **HEDGES, Steven, Kirk**
  **Fairfield, OH 45014 (US)**
- **EHRHARD, Joseph, Albert, Jr.**
  **West Chester, OH 45069 (US)**
- **SCHWARTZ, James, Robert**
  **West Chester, OH 45069 (US)**
- **WEISGERBER, David, John**
  **Cincinnati, OH 45239 (US)**

(74) Representative: **Brooks, Maxim Courtney et al**
**Rusham Park,**
**Whitehall Lane**
**Egham, Surrey TW20 9NW (GB)**

(56) References cited:
**EP-A- 0 308 190**
**US-A- 4 812 253**
**US-A- 5 262 079**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 752 846 B1

## Description

The present invention relates to personal skin moisturizing compositions and personal cleansing bar compositions.

## BACKGROUND OF THE INVENTION

Moisturizers are usually applied directly to the skin as leave-on products. Personal cleansing products are usually applied with water as a foam or lather and rinsed off with clear water. Ideal rinse off personal cleansers should cleanse the skin gently, causing little or no irritation without defatting and or drying the skin and without leaving skin taut after frequent use. Most lathering personal cleansing products, bar soaps, liquids and syndet bars fail in this respect.

Some current commercial personal cleansing bars claim to "moisturize" the skin. But, most of these current cleansing bar products do not deliver an adequate moisturizing benefit. Therefore, users typically must moisturize their skin with a separate leave-on product following cleansing. US-A-5 262 079 discloses a neutral pH, two-phase cleansing bar comprising 5-50% monocarboxylic acid, wherein 20-65% is neutralized, 20-65% of an anionic and/or nonionic bar firmness aid and from 15-55% water. US-A-4,812, 253 discloses air ultra mild skin cleansing composition comprising mild synthetic surfactants, moisturizers, polymeric skin feel and mildness aids and selected levels of soap. EP-A-308,190 discloses ultra mild toilet bars comprising 20-70% mild surfactant, 10-40% moisturizer 5.5-20% soap and 0.1-5% of a mixture of at least two polymeric skin feel and mildness aids.

It would be highly desirable to improve the delivery of skin moisturizers from a cleansing bar composition over the current commercial personal cleansing bars. If this were accomplished it would provide users with the convenience of obtaining both a cleansing and a moisturizing benefit from a single product.

Dual cleansing and lipid moisturizing bar compositions are very difficult to formulate and process. One reason is the cleansing ingredients, in general, tend to be incompatible with the lipid moisturizing ingredients. Another problem is processing, particularly, processing on a commercial scale, for they can be very sticky to process. Yet another problem is getting the lipid in the bar to deposit on the skin of the user . The deposition of lipid moisturizer from the bar, onto the skin can be very low due to loss of the lipid in the wash. Conversely, it can be too sticky if deposited on the skin. Still another problem is formulating a dual bar that lathers well. Another problem is formulating a dual bar that is hard and solid.

Needless to say, the actual deposition of lipid moisturizer from a lathering dual bar composition is essential for effective lipid benefit. No known prior art bar on the market today which claims to be a cleansing and lipid moisturizing bar, deposits as much as 3 micrograms of lipid moisturizer per cm. sq. of washed skin.

In conclusion, there has been a need for a dual cleansing and lipid moisturizing bar composition: 1) which produces an abundant, stable, high quality lather, 2) which is an effective skin cleanser, 3) which is very mild to the skin and ocular mucosae, 4) which actually delivers an effective amount of a lipid moisturizing agent to the skin of the user during the wash; and 5) which is processable.

It is an object of the present invention to provide an effective, yet gentle, dual skin cleansing bar composition which actually deposit a lipid on the skin to provide a skin moisturizing benefit while maintaining its lathering, sensory and cleaning properties.

## SUMMARY OF THE INVENTION

The present invention relates to a personal skin moisturizing and cleansing bar composition which comprises both a skin cleansing agent and a lipid moisturizing agent in the same bar which actually deposits an effective amount of the lipid on the skin of the user in the bath or shower.

According to the present invention there is provided a lathering skin cleansing composition comprising by weight parts of the bar composition:

(a) 5 parts to 40 parts of a lipid skin moisturizing agent, selected from the group of organic lipids, which are hydrophobic as defined by having a combined Vaughan Solubility Parameter (VSP) of from 5 to less than 10, and mixtures thereof,

(b) from 15 parts to 40 parts of a rigid semi-continuous, interlocking open mesh crystalline network structure consisting essentially of a fatty acid soap material which is a mixture of fatty acid soap and fatty acid, said fatty acid soap material having at least 75% saturated alkyl chains, said alkyl chains being selected from chains of from 8 to 22 carbon atoms, and mixtures thereof, wherein said soap and said fatty acid are present in a ratio of from 2:1 to 10,000:1; and;

(c) from 1 part to 50 parts of a lathering synthetic surfactant, and;

(d) from 10 parts to 50 parts water;

wherein said water and said lipid are predominantly within interstices of said open mesh crystalline network; wherein said bar composition has a Lipid Deposition Value of 3 to 1000.

The bar of the present invention can provide good cleansing, lather and good sensory feel and yet surprisingly provide a lipid moisturizing benefit via deposition of the lipid on the skin of the user. The bar composition is solid and on a macro scale is homogeneous.

## BRIEF DESCRIPTION OF DRAWINGS

Figures 1-5 are photographs which show magnified views of samples of bars of the present invention.

## DETAILED DESCRIPTION OF THE INVENTION

The present bar is a lathering skin cleansing bar composition comprising by *weight parts of the following bar composition*:

(a) from 5 parts to 40 parts of a lipid skin moisturizing agent, selected from organic lipids, which are hydrophobic as defined by having a combined Vaughan Solubility Parameter (VSP) of from 5 to less than 10; and mixtures thereof;

((b) from 15 parts to 40 parts of a rigid semi-continuous, interlocking open mesh crystalline network structure consisting essentially of fatty acid soap material which is a mixture of fatty acid soap and fatty acid; wherein said fatty acid soap material has at least 75 % saturated alkyl chains; said alkyl chains being selected from chains of from 8 to 22 carbon atoms, and mixtures thereof; and wherein said soap and said fatty acid have a ratio of from 2:1 to 10,000:1, preferably 2:1 to 200:1;

c) from 1 part to 50 parts of a lathering synthetic surfactant having an equilibrium surface tension value of from 15 to 50 dynes per cm as measured at the CMC at 25°C; and

(d) from 10 parts to 50 parts water;

wherein said water and said lipid are predominantly within interstices of said open mesh crystalline network; wherein said bar composition has a Lipid Deposition Value of 3 microgram to 1000 micrograms per sq. cm. of skin as measured by Lipid Deposition Protocol 1.

## GLOSSARY OF TERMS AS USED HEREIN:

Vaughan Solubility Parameter (VSP) is a calculated parameter used to define a lipid's solubility. Vaughan parameters typically have a range of 5-25.

Lipid Deposition Value (LDV) is a measure of how much lipid is deposited on skin from compositions herein, the reading corresponds to the amount measured using a Sebumeter (typically the mean of six readings), as defined in Lipid Deposition Protocol 1, herein.

Equilibrium Surface Tension is a measure of surface tension of a surfactant as measured at the critical micelle concentration at 25°C; units are dynes/cm.

Consistency, k, is a measure of lipid viscosity, used in combination with Shear index, to define viscosity for materials whose viscosity is a function of shear. The measurements are made at 35°C and the units are poise (equal to 100 cps).

Shear index, n, is a measure of lipid viscosity, used in combination with Consistency, to define viscosity for materials whose viscosity is a function of shear. The measurements are made at 35°C and the units are dimensionless.

Elastic Modulus G' is used to define rheological properties of lipid and is a measurement of a lipids ability to store or return energy. The measurements are made at 35°C and the units are dynes/sq. cm.

Viscous Modulus G" is used to define rheological properties of lipid and is a measurement of unrecoverable energy. The measurements are made at 35°C and the units are dynes/sq. cm.

All parts, percentages and ratios used herein are by weight basis and all measurements are at 25°C, unless otherwise indicated.

## DETAILED DESCRIPTION OF DRAWINGS

Figures 1-5 are photographs which show magnified views of samples of bars of the present invention. Cryo-Scanning Electron Microscopy (Cryo-SEM) sample preparation involves fracturing a shaped solid bar with simple pressure to obtain a fresh surface for examination. The fractured sample is reduced in size (razor blade) to approximately 1 mm x 1 mm x 5 mm. The sample is then frozen in liquid nitrogen and then mounted and coated with a thin platinum layer. The Cryo-SEM used is a Model S-4100 Hitachi Field Emission Cryo-SEM.
Figure 1 shows a fractured cross section of soap bar (Example X) at 20,000 X magnification, focused on a non-lipid section of the bar.
Figure 2 shows a fractured cross section of soap bar (Example X) at 2,000 X magnification, focused on the lipid droplets in the bar (3 to 10 microns in size).
Figure 3 shows a fractured cross section of soap bar (Example X) at 100 X magnification.
Figure 4 shows a fractured cross section of soap bar (Example Y) at 5,000 X magnification, focused on the aqueous and lipid sections. The lipid is present as a lipid in water emulsion.
Figure 5 shows a fractured cross section of soap bar (Example Y) at 200 X magnification.
The following numbers identify elements and details of the Figures.

1. Fractured cross section of a preferred lipid soap bar Example X.
2. Shows a continuous aqueous phase at 20,000 X.
3. Shows crystalline soap fibers at 20,000 X.
4. Shows lipid droplets of 3 to 10 microns in diameter at 2,000 X.
5. Shows a continuous aqueous phase at 2,000 X.
6. Shows air pockets at 100 X.
7. Shows a typical section of fractured bar at 100 X, around air pocket; this area is further magnified in Figures 1 and 2.
8. Shows a pitted area at 5,000 X, which is a continuous aqueous phase containing an emulsion.
9. Shows acyl Isethionate surfactant crystal platelets at 5,000 X.
10. Shows lipid droplets at 5,000 X.
11. Shows air pockets at 200 X.
12. Is a lower magnification (200 X) of the section type shown in Figure 4.

## THE LIPID SKIN MOISTURIZING AGENT

The lipid skin moisturizing agent in the bar composition provides the skin of the user with a moisturization benefit via deposition of the lipid on skin during use. In this invention the lipid skin moisturizing agent is defined with scrutiny. The lipid type and its physical properties in this present invention hold the key to the overall product effectiveness, and is restricted to a hydrophobic material with the following defined physical and rheological properties.

### Vaughan Solubility Parameter Value (VSP)

The lipid in this present invention is further defined by its solubility parameter, as defined by Vaughan in Cosmetics and Toiletries, *Vol. 103, p47-69, Oct. 1988.* A lipid having a Vaughan Solubility Parameter Value (VSP) of from 5 to 10, preferably 6 to 9.5 or less than ten (10) is preferred for use in the bar compositions herein.

| VAUGHAN SOLUBILITY PARAMETER TABLE* | |
|---|---|
| Cyclomethicone | 5.92 |
| Squalene | 6.03 |
| Petrolatum | 7.33 |
| Isopropyl Palmitate | 7.78 |
| Isopropyl Myristate | 8.02 |
| Castor Oil | 8.90 |
| Cholesterol | 9.55 |

* As reported in Solubility. Effects in Product, Package, Penetration and Preservation. C. D. Vaughan, Cosmetics and Toiletries, Vol. 103, October 1988.

Fatty acids, fatty acid soaps and water soluble polyols are specifically excluded from our definition of a lipid.

Thus stearic acid, glycerine and propylene glycol are excluded from our definition of a lipid.

## SOME PREFERRED LIPIDS

Notwithstanding the rheological and solubility requirements, a wide variety of lipid type materials and mixtures of materials are suitable for use in the compositions of the present invention. Preferably, the lipid is selected from the group consisting of hydrocarbons oils and waxes, silicones, fatty acid derivatives, cholesterol, cholesterol derivatives, di and tri-glycerides, vegetable oils, vegetable oil derivatives, and acetoglyceride esters, alkyl esters, alkenyl esters, lanolin and its derivatives, milk -tri-glycerides, wax esters, beeswax derivatives, sterols and phospholipids mixtures thereof.

Hydrocarbon oils and waxes: Some examples are petrolatum, mineral oil micro-crystalline waxes, polyalkenes, paraffins, cerasin, ozokerite, polyethylene and perhydrosqualene.

Silicone Oils: Some examples are dimethicone copolyol, dimethylpolysiloxane, diethylpolysiloxane, high molecular weight dimethicone, mixed C1-C30 alkyl polysiloxane, phenyl dimethicone, dimethiconol, and mixtures thereof More preferred are non-volatile silicones selected from dimethicone, dimethiconol, mixed C1-C30 alkyl polysiloxane, and mixtures thereof. Nonlimiting examples of silicones useful herein are described in US-A-5,011,681 to Ciotti et al., issued April 30, 1991.

Di and tri-glycerides: Some examples are castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil and sesame oil, vegetable oils and vegetable oil derivatives; coconut oil and derivatized coconut oil, cottonseed oil and derivatized cottonseed oil, jojoba oil, cocoa butter, and the like.

Acetoglyceride esters are used and an example is acetylated monoglycerides.

Alkyl esters can be used and some examples are: isopropyl esters of fatty acids and long chain esters of long chain fatty acids, e.g. cetyl ricinoleate are especially useful herein. Some examples of these are isopropyl palmitate, isopropyl myristate, cetyl riconoleate and stearyl riconoleate. Other examples are: hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate and cetyl lactate.

Alkenvl esters are useful and some examples are oleyl myristate, oleyl stearate and oleyl oleate.

Lanolin and its derivatives are preferred and some examples are lanolin, lanolin oil, lanolin wax, lanolin alcohols, lanolin fatty acids, isopropyl lanolate, acetylated lanolin, acetylated lanolin' alcohols, lanolin alcohol linoleate, lanolin alcohol riconoleate.

Milk glycerides are useful and an example is hydroxylated milk glyceride.

Polvol fatty acid polyesters are also useful.

Wax esters, such as beeswax and beeswax derivatives, spermaceti, myristyl myristate, stearyl stearate are also useful. Vegetable waxes are useful and some examples are carnauba and candelilla waxes. Sterols are useful and some examples are cholesterol, cholesterol fatty acid esters. Phospholipids, such as lecithin and derivatives, Sphingo lipids, ceramides, glycosphingo lipids are also useful.

It is more preferred when at least 70 % of the lipid is selected from: petrolatum, mineral oil micro-crystalline waxes, paraffins, ozokerite, polyethylene, polybutene, polydecene and perhydrosqualene. dimethicones, cyclomethicones, alkyl siloxanes, polymethylsiloxanes and methylphenylpolysiloxanes, lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol riconoleate castor oil, soy bean oil, maleated soy bean oil, safflower oil, cotton seed oil, corn oil, walnut oil, peanut oil, olive oil, cod liver oil, almond oil, avocado oil, palm oil and sesame oil, and mixture thereof.

It is most preferred when at least 70 % of lipid is composed of lipids selected from: petrolatum, mineral oil, paraffins, polyethylene, polybutene, polydecene, Dimethicones, alkyl siloxanes, cyclomethicones, lanolin, lanolin oil, lanolin wax. The remainder of the lipid is preferably selected from: isopropyl palmitate, cetyl riconoleate, octyl isononanoate, octyl palmitate, isocetyl stearate, hydroxylated milk glyceride and mixtures thereof.

The lipid is preferably in the bar as an emulsion having droplets ranging from about 0.1 microns to 100 microns, excluding anomalous very small or a few very large particles. While not being bound by any theory, particle size can impact the user perceived stickiness; with larger particles being perceived as more sticky. An example of a sticky lipid bar with large lipid particles is disclosed in allowed U.S. Pat 5312559, Kacher et al., filed July 7, 1992.

Pure petrolatum can be a sticky lipid and when used as the sole lipid in a 2-in-1 bar, it is preferred to have at least 75%, preferably 85%, of its particles smaller than 10 microns. However, when petrolatum is combined with other selected lipids the overall lipid stickiness can be reduced and the particle size for feel is less important and can range from 0.1 microns to 100 microns.

While not being bound by any theory, lipids outside of the rheology properties defined herein are either too easily emulsified and hence will not deposit, or are too "stiff" to adhere or deposit on to skin and provide a moisturization

benefit. The lipid rheological properties are considered to have an important effect on lipid deposition. In addition, the rheological properties of the lipid are also important to user perception. Some lipids, on deposition to the skin, are considered too sticky and are not preferred by the user.

| Lipid Rheological Table 1 | | | | |
|---|---|---|---|---|
| Range | k | n | G' at 1 Hz | G" at 1 Hz |
| | poise (1/sec)n-1 | (dimensionless) | (dynes/cm2) | (dynes/cm2) |
| Most preferred 100,000 | 50-2,000 | 0.20-.5 | 5,000-50,000 | 5,000- |
| More Preferred | 10-3,000 | 0.1-0.5 | 1,000-80,000 | 500-300,000 |
| Preferred | 5-5000 | 0.1-0.9 | 25-100,000 | 25-500,000 |

Two types of rheological parameters are used to define the lipid used herein. The viscosity of the fluid is represented by consistency (k) and shear index (n) and, while not being bound by any theory, is believed to represent the stickiness. The other type of parameter used herein, are the elastic modulus (G') and the viscous modulus (G"). While not being bound by any theory it is believed G' and G" are important factors determining the lipid's emulsification characteristics.

The useful lipid herein has a shear index, n, of from about 0.1 to about 0.8 and a consistency, k, of: from 5 to 5,000 poise; preferably 10 to 3000 poise; more preferably 50 to 2,000 poise at 35° C. The preferred lipid rheology is further defined in the following table:

The shear index, n, and consistency, k, are well accepted industry standards for reporting the viscosity profile of a material that has a viscosity that is a function of the shear rate.

For all materials the viscosity, which is defined for instance in "Chemical Engineering, by Coulson and Richardson" is given by:

Viscosity,

$$\mu = \sigma/\gamma'$$

Where $\sigma$ is the shear stress, and $\gamma'$ is the shear rate.

The viscosity for all materials is measured by either applying a shear rate and measuring the resultant shear stress or vice versa.

The Carrimed CSL 100 Controlled Stress Rheometer is used to determine Shear Index, n, and Consistency, k, for the lipids herein. The determination is performed at 35°C with the 4 cm 2° cone measuring system typically set with a 51 micron gap and is performed via the programmed application of a shear stress (typically from about 0.06 dynes/sq. cm to about 5,000 dynes/sq. cm) over time. If this stress results in a deformation of the sample, i.e. strain of the measuring geometry of at least 10-4 rad/sec, then this rate of strain is reported as a shear rate. These data are used to create a viscosity $\mu$ Vs. shear rate $\gamma'$ flow curve for the material. This flow curve can then be modeled in order to provide a mathematical expression that describes the material's behavior within specific limits of shear stress and shear rate. These results were fitted with the following well accepted power law model (see for instance: Chemical Engineering, by Coulson and Richardson, Pergamon, 1982 or Transport Phenomena by Bird, Stewart and Lightfoot, Wiley, 1960):

Viscosity,

$$\mu = k\,(\gamma')^{n-1}$$

## Lipid Rheological Table 2

| Lipids | Consistency, k | shear index | G' at 1 HZ | G" at 1 Hz |
|---|---|---|---|---|
| Units | poise | n | dynes/sq. cm | dynes/sq. cm |
| Water | 0.01 | 1.0 | | |
| Microcrystalline Wax (MC) | ** | ** | ** | ** |
| 80 % Pet/20 % MC wax | 3926-4822* | 0.31-33* | 306,400-621,000* | 434,000-594,580* |
| 91 % Pet/9 % MC Wax | 1983 | 0.15 | | |
| Petrolatum | 1080-1345 | 0.24 | 25,000-40,000 | 23,400-36,400 |
| 90 % Pet/10 % min oil | 767-780 | 0.26 | | |
| 80 % Pet/20 % min oil | 354-430 | 0.29-0.34 | 8,500-9300 | 6,700-7000 |
| 60 % Pet/40 % min oil | 111-115 | 0.42 | 1,000-2800 | 940-2500 |
| 40 % Pet/60 % min oil | 4.8-5.3 | 0.87 | 230-380 | 280 |
| Mineral (min) oil | 0.81-0.82 | 1.0 | | |
| 5 %SE■/95 % min oil | 1580-1787 | 0.16 | | |
| 95.9 %SBO/4.1 %MC wax | 780-890 | 0.13-0.16 | | |
| 80 % Pet/20 % Polydecene | 283-292 | 0.32-0.34 | 5881-7160 | 6118-6805 |
| 65 % Pet/35 % Polydecene | 115-120 | 0.4 | 1280-1407 | 1416-1446 |
| 20 % Pet/80 % Polydecene | 0.83 | 0.97-1.0 | 24.1 | 34.5 |
| 20 % SE■/80 % Polydecene | 1897-2035 | 0.19-0.22 | 1E6-1,370,000 | 280,000-980,000 |

* Measured with same instrument, but with 2 cm parallel plate geometry.

** Too stiff and solid to obtain readings

■ SE solid is a sucrose ester solid and is an example of a preferred polyol fatty acid polyester. SBO is soybean oil and Pet is petrolatum.

Note that mineral oil, microcrystalline wax and some other lipids by themselves have rheological properties that are unsuitable for use in the present bar compositions; but may be blended with other lipids to provide acceptable lipid blends.

## Test Protocol for determination of G' and G"

The Carrimed CSL 100 Controlled Stress Rheometer is used to perform oscillatory tests at 35°C with the 4 cm 2° cone measuring system typically set with a 51 micron gap. The oscillatory tests at 35°C are carried out in 2 steps. The first step is a stress amplitude sweep at the expected starting and ending frequencies for the frequency sweep. These tests allow a determination to be made as to whether or not the test conditions are within the linear viscoelastic region for the test material over the anticipated frequency range. The linear viscoelastic region is a region where there is a linear relationship between stress and strain. The second step is a frequency sweep made at a stress level within that linear viscoelastic region. The frequency sweep allows the test material's viscoelastic behavior to be measured. The oscillatory test on a controlled stress rheometer is performed by applying a stress in an oscillatory manner and measuring the resulting oscillatory strain response and the phase shift between the applied stress wave form and the resulting strain wave form in the test material. The resulting complex modulus is expressed as a combination of the material's elastic (G') and viscous (G") components:

The elastic modulus G' is a measure of a materials ability to store recoverable energy. This energy storage

can be the result of the ability of a complex polymer, structural network, or a combination of these to recover stored energy after a deformation. The viscous or loss modulus G" is a measure of the unrecoverable energy which has been lost due to viscous flow.

The lipid is present in the bar at a level of from 5 parts to 40 parts by weight of the bar. Its more preferred levels are 10 parts to 40 parts; and 10 parts to 30 parts. The most preferred lipid levels are from 12 or 15 parts to 25 parts.

Known market bars contain little or no lipid. Known market bars that contain lipid deposit lipid at an efficiency of less than 3 microgram per sq. cm. of skin as measured by Deposition Protocol 1.

The lipid in this present invention is deposited on skin during use at an efficiency that produces at least 3 µg of lipid per sq. cm of skin. The preferred level of deposition is from 5 µg/sq. cm to 1000 µg/sq. cm. The more preferred levels are from 5 or 10 µg/sq. cm to 500 µg/sq. cm and 25 µg/sq. cm to 500 µg/sq. cm, as measured by lipid Deposition Protocol 1. It has been found that a certain minimum level of lipid is required in order to get any measurable deposition of the lipid on skin.

## LIPID DEPOSITION VALUE

The level of lipid deposition on skin can be measured by different protocols, all are modeled after how skin cleansing products are typically used by consumers. All the protocols are "in vivo", and all tests are made using a statistically designed protocol using at least 6 subjects per prototype.

All protocols consist of a common exaggerated product application stage followed by a determination of the deposited lipid amount. The following two protocols only differ in the analytical technique used to quantify the amount of deposited lipid on the skin. The quantification of lipid is "in vivo" and as such has a wide variance due to differences in skin type and condition. To offset this a balanced design is used to test prototypes; balanced in skin type and using a large base size. In all cases product application and measurement is undertaken by a trained technician to reduce variability.

### Prep For Lipid Deposition For Protocols 1 & 2

The subject wets the entire surface of the inner forearm with 95-100°F (35° C-38°C) water. The technician using exam gloves, wets the appropriate bar with tap water and then rotates the bar in both hands for (10) seconds to generate lather. The technician then rubs the bar on the inner forearm from the wrist to the elbow and back down again for 20 seconds (i.e., exactly 20 rubs up and 20 rubs down). The bar is then set aside and the subject's forearm is rubbed by the technician for ten seconds by rubbing the gloved hand up and down the subject's inner forearm, again from elbow to wrist. The lather is allowed to remain on the forearm for fifteen seconds, followed by a thorough rinse for fifteen seconds. After rinse, the technician gently pats the forearm dry with a disposable paper towel. The process is repeated two more times for a total of three washes.

## LIPID DEPOSITION PROTOCOL 1

The unit used is a Sebumeter SM810 which is commercially available from Courage and Khazaka GmbH and is reported to be recognized by the scientific world. The Sebumeter measures lipid on the skin via photometry of a special plastic strip, which becomes transparent when it absorbs lipids. The plastic strip is extended over a mirror which is connected to a spring. The measuring head of the device (comprised of spring, mirror and plastic strip) is pressed against the skin for 30 seconds. The value (µg/sq. cm) is indicative of the amount of lipid on the skin, and increases with increased amount of lipid. The method is insensitive to humidity. Six Sebumeter readings are taken along the length of the forearm and the Lipid Deposition Value, LDV, (µg/sq. cm) is defined as the mean of the six readings.

The Sebumeter has the following limitations:

1. The Sebumeter tape also detects natural skin lipids. A criterion of this test was that subjects baseline value measured on the Sebumeter, prior to washing, be less than or equal to 1 or 2 µg/sq. cm of forearm skin.
2. The Sebumeter like other surface extraction measurements may not measure all the deposited lipid, if the skin topography is undulating it is possible that deposited lipid may not be extracted by the Sebumeter tape.
3. The Sebumeter tape becomes saturated at a LDV of above about 300 µg/sq. cm; so it is understood that for deposition values above 300 µg/sq. cm, Protocol 2 is used.

## LIPID DEPOSITION PROTOCOL 2

The second protocol uses a solvent extraction method similar in type to that described in the Journal Society of Cosmetic Chemists of Great Britain *Vol*. 21 (p *521-532)*, *1970*. An extraction cup is firmly attached to the forearm

and heptane poured in to the cup, such that it is in contact with the forearm. The solvent extract containing the extracted lipid is analyzed by standard gas chromatographic methods.

## LIPID DEPOSITION VALUE CONVERSION

Lipid Deposition Values, LDV, (as defined by the mean of six Sebumeter Readings using Protocol 1) may be converted into actual deposition of lipid on skin. This conversion factor is dependent on lipid type. For example, a petrolatum deposition of about 5 µg/sq. cm for Protocol 2 is equal to a Sebumeter Lipid Deposition Value of about 2 µg/sq. cm and a petrolatum deposition of about 90 µ g/sq. cm for Protocol 2 is equal to a Sebumeter Lipid Deposition Value of about 52 µg/sq. cm.

## THE RIGID CRYSTALLINE NETWORK STRUCTURE

The rigid crystalline network structure is a crystalline skeleton structure comprising a rigid interlocking, open, three-dimensional mesh which consists essentially of the selected fatty acid soap material. The fatty acid soap material is a mixture of said soap and selected fatty acid. The crystalline network can comprise crystals in the form of either interlocking platelets and/or fibers, preferably fibers. Preferably said fibers are composed of sodium and magnesium soap and most preferable sodium soap. The interlocking mesh can impart strength to the three-dimensional structure, even in the presence of relatively high levels of lipid and water; and even when the bar is allowed to soak overnight in water.

Once formed, a bar (shaped solid) comprising the rigid skeletal structure of the present invention loses its rigidity when subjected to fracturing mechanical forces, e.g., those used in a conventional plodded bar making process as disclosed in U.S. Pat. No. 4,812,253, Small et al., or U.S. Pat. No. 4,820,447, Medcalf et al. While not being bound to any theory, it is theorized that this is because the fracturing mechanical forces shear and break up the rigid, skeletal structure into smaller pieces. Thus, when a bar composition of the present invention is flaked or the finished bar sheared in a plodder, a much softer bar results.

On the other hand, when a finished conventional bar is plodded or replodded, the replodded conventional milled bar or freezer bar is still very hard.

The skeletal structure contains substantial "void" areas which are filled by a two phase lipid and aqueous emulsion, preferably an oil in water emulsion. The presence of said rigid crystalline structure is believed to be a necessary, but not the only condition for lipid deposition from a 2-in-1 bar. Some bars which contain lipid in their formulation and which have rigid crystalline structure, do not deposit at least 3 µg of lipid per sq. cm of skin tested using Lipid Deposition Protocol 1. One such bar is made by a freezer process as disclosed in US Pat. 5425892 to Taneri et al., and is distinguished from the bars of this invention.

It is a surprising aspect of this invention that a substantial level of lipid and water, which can form an oil in water emulsion, can be incorporated into a solid bar without having a significant negative impact on the physical properties of the bar, such as bar hardness, lather and smear; and that said bar can deposit at least 3 µg of lipid, per square centimeter of skin, as measured using Lipid Deposition Protocol 1. In conventional bars, high levels of water and lipid can impact the overall bar physical properties because the components either modify the phase and structure of the soap or synthetic surfactant components that primarily determine the bar's physical properties. The combination of two or more phases (e.g., soap and aqueous solution) drastically changes the colloidal structure, and consequently, the physical properties and processability of a conventional bar.

Thus, conventional bars are more limited in the type, levels and composition of materials that can be incorporated into the bar than the present invention.

The soap (neutralized carboxylic acid) is selected from sodium soap, magnesium soap and sodium di-carboxylic acid; and mixtures thereof. The monocarboxylic acid has a fatty alkyl chain of from 8 to 24 carbon atoms and the di-carboxylic acid has a fatty alkyl chain of from about 12 to 18 carbon atoms. The more preferred fatty acid soap material is defined herein as a mixture of monocarboxylic fatty acid soap and monocarboxylic fatty acid; wherein fatty acid soap material is at least 80 % saturated and has an alkyl chain of from 8 to 22 carbon atoms, and mixtures thereof. The most preferred fatty acid material is essentially saturated fatty acid alkyl chains of from 8 to 22 carbon atoms with at least 75 % of it having a chain length of from 10 to 18; preferably 12 to 18 carbon atoms and of which at least about 25 %, and most preferably 50 % of said saturated fatty alkyl chains have 12 to 14 carbon atoms.

In other words, at least 80 %, preferably 90 %, of the monocarboxylic acid (of the fatty acid material) has the following general formula:

$$H-(CH_2)_a-\underset{\displaystyle X}{\underset{|}{CH}}-(CH_2)_b-CO_2-M^+$$

wherein:

a+b= 8 to 20
each a, b = 0 to 20
X = H, OR, O-CO-R, R, or mixtures thereof
R = C1-C3 alkyl, H, or mixtures thereof
M = Na, (½Mg), or mixtures thereof.

The above cleansing bar is more preferred when said a + b = 10-16; each of said a, b = 0-16; said X = H, OR; R = H; and M = Na. Examples of the most preferred fatty acids are: lauric acid, myristic acid, palmitic acid, stearic acid and 12 -hydroxystearic acid.

The fatty acid soap material of the present invention comprises 15 parts to 40 parts by weight of the bar. Some preferred levels of fatty acid soap material are from 15 parts to 35 parts and from 15 parts to 25 parts *by weight of the bar.*

A bar is preferred when the fatty acid soap material is sodium and magnesium fatty acid soap, and is highly preferred when no more than 25 % is magnesium soap.

The ratio of said unneutralized (free) carboxylic acid to soap is from 1:2 to 1:10,000, and a most preferred ratio is from about 1:4 to 1:10,000. Some other ratio examples are: 1:10 and 1:200.

The following Table set out some levels and preferred properties of said fatty acid soap material which forms said rigid crystalline network structure.

| Fatty Acid Soap Material Table | | | |
|---|---|---|---|
| | Broad | Preferred | Most |
| preferred Fatty Acid Soap parts | 10-50 parts | 15-35 parts | 15-25 parts |
| Chain Length | 8-24 | 8-22 | 12-18 |
| % saturated | 75 % | 80 % | 90 % |
| Minimum ratio of Soap:Fatty Acid* | 1:3 | 2:1 | 4:1 |

*All soap bars can be made with little or no free fatty acid.

## THE LATHERING SYNTHETIC SURFACTANT

The bar composition comprises a lathering synthetic surfactant selected from anionic surfactants; nonionic surfactants, cationic surfactants, amphoteric surfactants, and mixtures thereof.

The lathering synthetic surfactant is defined herein as a synthetic surfactant or mixes thereof that when combined have an equilibrium surface tension of between 15 and 50 dynes/cm, more preferably between 20 and 45 dynes/cm as measured at the CMC (critical micelle concentration) at 25°C. Some surfactant mixes can have a surface tension lower than those of its individual components.

| **TABLE OF SOME SYNTHETIC SURFACTANTS SURFACE TENSION*** | |
|---|---|
| Surfactant | Surface tension at CMC |
| (dynes/cm) Anionics | |
| Sodium Dodecane Sulfonate | 43 |
| Potassium Dodecane Sulfonate | 38 |
| Sodium Dodecyl Sulfate | 40 |
| Sodium Tetradecyl Sulfate | 35 |
| Sodium hexadecyl Sulfate | 37 |
| Sodium Dodeceth-2 Sulfate | 42 |

* As calculated from Surfactants and Interfacial Phenomena by Rosen, Wiley, 1988)

(continued)

| TABLE OF SOME SYNTHETIC SURFACTANTS SURFACE TENSION* | |
|---|---|
| Surfactant | Surface tension at CMC |
| (dynes/cm) Anionics | |
| Sodium Decyl Benzene Sulfonate | 48 |
| Sodium Dodecyl Benzene Sulfonate | 47 |
| Sodium Hexadecyl Benzene Sulfonate | 45 |
| Cationics | |
| Tetradecyl Trimethyl Ammonium Bromide | 41 |
| Dodecyl Trimethyl Ammonium Methane Sulfonate | 39 |
| Zwitterionics | |
| Dodecyl Betaine | 33 |
| Hexadecyl Betaine | 35 |
| Dodecyl Benzyl methyl Ampho Acetate | 33 |
| Nonionics | |
| 1,2 Dodecyldiol | 23 |
| 1,3 Pentadecyldiol | 27 |
| Hexeth-6 | 32 |
| Deceth-6 | 30 |
| Dodeceth-3 | 28 |
| Dodeceth-12 | 40 |
| Hexadeceth-6 | 32 |
| Hexadeceth-21 | 45 |
| Nonoxynol-10 | 31 |
| Nonoxynol-30 | 41 |
| Dimethicone copolyol | 21-22 |

* As calculated from Surfactants and Interfacial Phenomena by Rosen, Wiley, 1988)

| TABLE OF SOME PREFERRED SURFACTANTS SURFACE TENSION** | |
|---|---|
| Surfactant | Surface tension (dynes/cm) |
| C12-C14 Glycerylether sulfonate | 47 |
| Sodium Lauryl Isethionate | 42 |
| Sodium Coco Isethionate | 42 |
| Sodium Stearyl Isethionate | 72 |
| Sodium Ether (3) Sulphate | 47 |
| Sodium Coco Taurate | 43 |
| Sodium Lauryl Sarcosinate | 42 |

**Measured on Kruss BP-10 Dynamic surface tensiometer, these measurements were not equilibrium, por at the CMC. Equilibrium measurements are typically lower than Dynamic.

The combined personal cleansing and moisturizing bar composition herein comprises at least from 0.5 or 1 part to 50 parts, preferably from 5 parts to 40 parts, and most preferably from 10 parts to 35 parts of a lathering synthetic surfactant.

Anionic surfactants useful herein include acyl isethionates, acyl sarcosinates, alkylglycerylether sulfonates, methylacyl taurates, paraffin sulfonates, linear alkyl benzene sulfonates, N-acyl glutamates, alkyl sulfosuccinates, alpha sulfo fatty acid esters, alkyl ether carboxylates, alkyl phosphate esters, ethoxylated alkyl phosphate esters, alpha olefin sulphonate, the alkyl ether sulfates (with 1 to 12 ethoxy groups), and mixtures thereof, wherein said surfactants contain C8 to C22 alkyl chain. The anionic surfactant is more preferred at 8 parts to 30 parts, selected from acyl isethionate, acyl sarcosinates, alkyl sulfosuccinates, alkylglycerylether sulfonates, methylacyl taurates, alkyl ether sulfates, alkyl sulfates, alkyl phosphate esters and mixtures thereof, wherein said surfactants contain C8 to C18 alkyl chains and wherein the counterion is selected from Na, K, $NH_4$, $N(CH_2CH_2OH)_3$.

Amphoteric synthetic surfactants cannot serve as the sole surfactant in this product, but are preferred as a co-surfactant at a lower level of from 1 part to 10 parts, by weight and the more preferred types are selected from alkylampho mono- and di-acetates, alkyl betaines, alkyl sultaines, alkyl amidopropyl betaines, alkyl amidopropyl hydroxysultaines, and mixtures thereof, wherein said surfactants contain C8 to C22 alkyl chains.

Nonionic synthetic surfactant cannot serve as the sole surfactant in this product, but can be used as a co-surfactant at a lower level of from 3 parts to 17 parts , by weight. The more preferred types selected from alkyl glucose amides, alkyl glucose esters, polyoxyethylene amides, fatty alkane amides, alkyl amine oxides, alkyl polyglucosides, polyoxy ethylene alkyl phenols, polyoxyethylene esters of fatty acids, EO/PO block co-polymers such as polyoxamines and poloxamers, sorbitan esters and alcohol esters, and mixtures thereof.

Cationic synthetic surfactant cannot serve as the sole surfactant in this product, but are preferred as a co-surfactant at a lower level of from 0.5 parts to 6 parts, by weight. The more preferred types of cationic surfactants are selected from : alkyl trimonium chloride and methosulfate, and dialkyldimonium chloride and methyl sulphate, and alkyl alkonium chloride and methyl sulphate and mixtures thereof. These surfactants contain C12 to C24 carbon atoms per alkyl chain. The most preferred cationic is selected from stearalkonium chloride, stearyltrimonium chloride, Di-stearyldimonium chloride, and mixtures thereof.

Cationic surfactants may also act as a lipid deposition aid and thus a preferred lathering skin cleansing bar composition comprising b*y weight parts of the bar composition*:

(a) from 15 parts to 40 parts of a rigid semi-continuous, interlocking open mesh crystalline network structure consisting essentially of a fatty acid soap material which is a mixture of fatty acid soap and free fatty acid, said fatty acid soap material having at least 75% saturated alkyl chains; said alkyl chains being selected from chains of from 8 to 22 carbon atoms, and mixtures thereof; wherein said soap and said fatty acid are present is a ratio of from 2:1 to 10,000:1; and
(b) from 3 parts to 40 parts of a lipid skin moisturizing agent, selected from organic lipids, which are hydrophobic as defined by having a Vaughan Solubility Parameter (VSP) of from 5 to less than 10; and mixtures thereof;
(c) from 1 part to 50 parts of a lathering synthetic surfactant; and
(d) from 10 parts to 50 parts water,
(e) from 0.5 to 6 parts of a cationic surfactant; and

wherein said water and said lipid are predominantly within interstices of said open mesh crystalline network; wherein said bar composition has a Lipid Deposition Value of 3 microgram to 1000 micrograms per sq. cm. of skin as measured by Lipid Deposition Protocol 1; and wherein said cationic surfactant improves the Lipid Deposition Value.

WATER AND THE AQUEOUS PHASE

The moisturizing and cleansing bar compositions of the present invention comprise water as an essential component. The water is present at a level of from 10 parts to 50 parts, preferably from 12 parts to 45 parts, and most preferably from 15 to 35 parts. A substantial percentage of the water forms the key part of an aqueous phase, which may also contain other water soluble components. Polyols and surfactants are water soluble.

While not being bound to any theory, the presence of a lipid in water emulsion is believed to be important to lipid deposition on the skin. The level of water is key to forming a lipid in water emulsion. Thus, an effective amount of water is required to form an aqueous phase to support the lipid in water emulsion. The level of aqueous phase to lipid is preferably greater than 1:1, e.g., 2:1.

While not being bound to any theory, the interlocking crystalline network physically entraps the lipid in water emulsion, preventing phase separation of the lipid. On lathering the emulsion is released. It has also been found that bars of the compositions herein, produce a lipid in water emulsion in its lather. This emulsion in the lather is unstable and deposits lipid on the skin during the wash cycle.

The upper range of water is adjusted to provide a desired bar hardness and bar composition stability. Also enough water is required to properly process the bar, so the lower amount of water is restricted by an ability to pour the mix, comprising the final bar composition, into molds at the pour temperature and yet provide a bar which provides lipid deposition.

**Optional Ingredients**

A highly preferred optional component of the present compositions are one or more humectants and solutes. A variety of humectants and solutes can be employed and can be present at a level of from about 0.1 % to 50 %, more preferably from 0.5 % to 35 %, and most preferably from 2 % to 20 %. of a non-volatile, organic material having a solubility of a least 5 parts in 10 parts water. A preferred water soluble, organic material is selected from a polyol of the structure:

$$R1 - O(CH_2 - CR2HO)_nH$$

where R1 = H, C1-C4 alkyl; R2 = H, $CH_3$ and n = 1 - 200; C2-C10 alkane diols; guanidine; glycolic acid and glycolate salts (e.g. ammonium and quaternary alkyl ammonium); lactic acid and lactate salts (e.g. ammonium and quaternary alkyl ammonium); polyhydroxy alcohols such as sorbitol, glycerol, hexanetriol, propylene glycol, hexylene glycol and the like; polyethylene glycol; sugars and starches; sugar and starch derivatives (e.g. alkoxylated glucose); panthenol (including D-, L-, and the D,L- forms); pyrrolidone carboxylic acid; hyaluronic acid; lactamide monoethanolamine; acetamide monoethanolamine; urea; and ethanol amines of the general structure $(HOCH_2CH_2)_xNH_y$ where x = 1-3; y = 0-2, and x+y = 3, and mixtures thereof. The most preferred polyols are selected from the group consisting of glycerine, polyoxypropylene(1) glycerol and polyoxypropylene(3) glycerol, sorbitol, butylene glycol, propylene glycol, sucrose, urea and triethanol amine.

Polyols may also act as a lipid deposition aid and a preferred lathering skin cleansing bar composition comprising b*y weight parts of the bar composition:*

(a) from 15 parts to 40 parts of a rigid semi-continuous, interlocking open mesh crystalline network structure consisting essentially of a fatty acid soap material which is a mixture of fatty acid soap and free fatty acid said fatty acid soap material having at least 75% saturated alkyl chains; said alkyl chains being selected from consisting of chains of from 8 to 22 carbon atoms, and mixtures thereof; wherein said soap and said fatty acid are present is a ratio of from 2:1 to 10,000:1; and
(b) from 3 parts to 40 parts of a lipid skin moisturizing agent, selected from organic lipids, which are hydrophobic as defined by having a Vaughan Solubility Parameter (VSP) of from 5 to less than 10; and mixtures thereof;
(c) from 1 part to 50 parts of a lathering synthetic surfactant; and
(d) from 10 parts to 50 parts water;
(e) from 0.5 to 35 parts water soluble polyol.

wherein said water soluble organic material is at least 50 % soluble in water; and wherein said water and said lipid are predominantly within interstices of said open mesh crystalline network; wherein said bar composition has a Lipid Deposition Value of 3 micrograms to 1000 micrograms per sq. cm. of skin as measured by Lipid Deposition Protocol 1. The polyol is used at a preferred level which improves the Lipid Deposition Value.

The above lathering skin cleansing bar composition is preferred when said water soluble organic material is from 3 parts to 20 parts, by weight of said bar; and wherein said organic material is selected from glycerine, polyoxypropylene (1) glycerol and polyoxypropylene (3) glycerol, sorbitol, butylene glycol, propylene glycol, sucrose, and urea and triethanolamine; and wherein said lipid is from 15 parts to 25 parts, by weight of the bar; and wherein said synthetic surfactant is from 15 to 30 parts by weight of the bar; said soap is present at a level from 15 parts to 25 parts and said soap is essentially C-12-18 carboxylic acids, with at least 50 % being C12 or C14; and wherein,at least 75 % of said lipid by weight of said lipid is selected from: petrolatum, micro crystalline wax, mineral oil or polydecene and mixes thereof; and wherein 5 to 25 % of said lipid by weight of said lipid is selected from: dimethicone or alkyl polysiloxane.

The use of oil thickening polymers, such as those listed in EP 0 547 897 A2 to Hewitt, published 23/06/93, are useful if the final rheology of lipid and polymer falls within the preferred range.

A preferred optional ingredient are one or more cationic and/or nonionic polymeric skin conditioning agents. A variety of polymers can be employed and can be present at a level of from 0.1 parts to 10 parts, and more preferably 0.25 parts to about 3 parts of a polymeric, nonionic, cationic or hydrophobically modified polymeric skin feel aid, selected from cationic polysaccharides of the cationic guar gum class with molecular weights of 1,000 to 3,000,000, cationic and nonionic homopolymers derived from acrylic and/or methacrylic acid, cationic and nonionic cellulose resins; cationic copolymers of dimethyldialkylammonium chloride and acrylic acid; cationic homopolymers of dimethyldialkylammonium chloride; cationic polyalkylene and ethoxypolyalkylene imines; and mixes thereof. Examples are hydroxypropyl guar, guar hydroxypropyltrimonium chloride, polyquaternary 3, 5, 6, 7, 10, 11 and 24. In order to achieve the benefits described in this invention, the polymer must have characteristics, either structural or physical which allow it to be suitably and fully hydrated and subsequently well incorporated into the soap matrix.

## Other Optional Components

A variety of additional ingredients can be incorporated into the compositions of the present invention. These materials including, but not limited to, bar appearance aids, salts and their hydrates, clays, and other "filler materials" are listed in US Pat. 5340492 to Kacher et al,. Examples of other suitable materials are disclosed in U.S. Patent No. 4,919,934, to Deckner et al., issued April 24, 1990;

Other non limiting examples of these additional ingredients include vitamins and derivatives thereof (e.g., ascorbic acid, vitamin E, tocopheryl acetate, and the like); sunscreens; thickening agents (e.g., polyol alkoxy ester, available as Crothix from Croda); preservatives for maintaining the anti microbial integrity of the compositions; anti-acne medicaments (resorcinol, salicylic acid, and the like); antioxidants; skin soothing and healing agents such as aloe vera extract, allantoin and the like; chelators and sequestrarits; and agents suitable for aesthetic purposes such as fragrances, essential oils, skin sensates, pigments, pearlescent agents (e.g., mica and titanium dioxide), lakes, colorings, and the like (e.g., clove oil, menthol, camphor, eucalyptus oil, and eugenol).

More preferably the composition will include from 0.1 to about 50 % of other ingredients selected from

- from 0.5 to 5 parts potassium soap;
- from 0.5 to 5 parts alkoxylated ammonium and/or alkylammonium and/or alkoxylated aliphatic amines and/or polyethoxylated amine soap;
- from 0.5 to 15 parts calcium soap;
- from 0.5 to 50 parts of impalpable water insoluble materials selected from calcium carbonate and talc;
- from 0.5 to 25 parts of aluminosilicate clay and/or other clays; wherein said aluminosilicates and clays are selected from zeolites, kaolin, kaolinite, bentonite, halloysite, calcined clays, montmorillonite, illite and attapulgite.
- from 0.5 to 25 parts dextrin, corn and wheat starch;
- from 0.1 to 15 parts of salt and salt hydrates; and mixtures thereof; and wherein said salt and salt hydrate have a cation selected from sodium, potassium, magnesium, calcium, aluminum, lithium ammonium, alkoxylated ammonium, alkylammonium, alkoxylated aliphatic amines, polyethoxylated amines; and wherein said salt and/or salt hydrate have an anion selected from the group consisting of: chloride, sulfate, isethionate, bromide, metasilicate, orthophosphate, pyrophosphate, polyphosphate, metaborate, tetraborate, carbonate, bicarbonate, hydrogen phosphate, methyl sulfate, and mono- and polycarboxylate of 1 to 6 carbon atoms or less;
- from 0.1 to 1 parts whitening aid;
- from 0.1 to 2 parts of a fragrance or perfume;
- from 0.1 to 5 parts of a chelant, preserve or anti fungal/anti microbial/bacterial agent.

Non limiting examples of suitable carboxylic copolymers, emulsifiers, emollients, and other additional ingredients are disclosed in U.S. Patent No., 5,011,681, to Ciotti et al., issued April 30, 1991,

## THE BAR COMPOSITION

As described above, the bar dual composition of this invention can provide good cleansing and foaming and yet moisturize the skin via lipid deposition. The bar composition of this invention itself has a Lipid Deposition Value (LDV) of at least 3 micrograms per sq. cm. This means that it will deposit at least 3 micrograms of lipid on a sq. cm of forearm skin using Lipid Deposition Protocol 1 disclosed herein.

While not being bound to any theory, the presence of an unstable lipid in water emulsion in the lather is believed to be key to deposition of lipid on the skin during the wash cycle.

## Two-in-One Bar Lipid Stability Centrifuge Test

The following test is used to determine the degree of stability of the lipid in a 2-in-1 (dual) bar composition. A ten percent (10 %) solution of the bar is made by weighing 10 grams of the bar, finely chopped, to 120°F (49 C) city water. It is stirred overnight (18 hours) using a magnetic stir plate and 1/2" stir bar.

The stirred sample is then centrifuged for 150 minutes at 10,000 RPM and 25°C using a Beckman L8-80-R60 Ultracentrifuge with a SW-40 rotor. If the lipid in water emulsion is suitably unstable, a lipid top layer is observed.

Such a sample prepared from a bar (Example B herein ) of this invention has a 1 mm sticky white top lipid layer, a 74 mm layer of clear liquid and a 1 mm white solid bottom layer.

An IR scan of the top layer shows the composition to be predominately petrolatum, with traces of water, sodium coco isethionate and glycerine present, confirming the emulsion model.

No known commercially available prior art bar which contains a lipid has a measurable quantity of lipid on the top layer, when subjected to the above test.

The dual moisturizing and cleansing bar of this invention can be made by either of the following processes:

**A BATCH PROCESS**

1. Fatty acid precursor(s) and polyol if used are heated to 70°C,
2. A salt, water (excluding water coming in with other raw materials) and caustic solution (50 % sodium hydroxide) are added and the mixture is stirred at a slow speed until smooth forming an aqueous molten liquid. The temperature during neutralization of the molten liquid increases to ~95°C.
3. The following ingredients are added preferably in the following order and the temperature is maintained at ~85°C: synthetic surfactant, preservatives (if any), whitening aid (if any) and some sensory aid, such as silicones. Perfume is the penultimate ingredient.
4. The pre-heated lipid, is added last and mixed for about two minutes on low speed. The temperature is maintained at about 85°C. the duration and intensity of the mix post lipid is considered important. If mixed too long or too little or too intense the bar quality or lipid deposition suffers. The goal is to create a uniform mix with lipid emulsion properties that yield bars which have lipid depositions values of from 2 to 500 micrograms per square centimeter.
5. The molten liquid mixture is poured into shaped molds. The molten liquid crystallizes (solidifies) on cooling to room temperature and the resultant bars are removed from the molds.

**AN IN-LINE MIXING PROCESS**

1. Fatty acid precursor(s) and optionally polyol are heated to about 70°C in Mixing Vessel 1,
2. A salt, water (excluding water coming in with other raw materials) and caustic solution (50 % sodium hydroxide) are added and the mixture is stirred at a slow speed until a smooth aqueous molten liquid is formed. The temperature during neutralization of the molten liquid increases to about 95°C.
3. Other ingredients are added preferably in the following order and the temperature is maintained at ~85°C: synthetic surfactant, preservatives (if any), whitening aid (if any) and some sensory aid, such as silicones Perfume is the penultimate ingredient.
4. The lipid is pre-heated to about 70-75°C in a separate Mixing Vessel 2
5. The material from Mixing Vessels 1 and 2 are pumped and metered together at pre-determined rates designed to yield the final composition. The combined stream passes through a cooling and agitating device unit (freezer) as described in US Patent No. 2,295,594, to Mills, issued Sept. 15, 1942 and 3,835,058 to White, issued Sept. 10 1974. which provides both partial cooling and mixing. Cooling in the present process is optional and must be limited. A pourable temperature is maintained and the duration and intensity of the mix for this step is considered very important. If mixed too long or too little or too intense the pourablity of the mix or the bar quality or the bar lipid deposition suffer. The goal is to create a uniform and pourable mix which has only semi-stable lipid emulsion properties and yield bars which are uniform and have lipid depositions values of from about 3 to 500 micrograms per square centimeter.
6. The uniform and pourab;e mixture exits the freezer via a nozzle and flows into bar shaped molds. The mix crystallizes (solidifies) on cooling to room temperature and the resultant bars are removed from the molds or the final mold acts as the package.

**Bar Lather Test**

The hand wash lather test is used to provide in-use lather volume measurements for the lather performance of skin cleansing bars. The test measures the lather volume generated under a soil load. Synthetic soil is used for the test reported herein. Its formula is reported in US 4,673,525 to Small et al. issued June 16th 1987.

**Bar Hardness Test**

1. The hardness of a bar is determined by measuring at 25°C the depth of penetration (in mm) into the bar of a 247 gram Standard Weighted Penetrometer Probe having a conical shaped needle attached to a 22.9 cm (9 inch) shaft weighing 47 grams with 200 grams on top of said shaft. A hardness measurement of 5 mm or less indicates a very hard bar; 5-12 mm indicates a moderately hard bar. This defines "hardness" as used herein unless otherwise specified.

**Bar Smear Test**

2. The smear grade is determined by a (1) placing a soap bar on a perch in a 1400 mm diameter circular dish; (2) adding 200 ml of room temperature water to the dish such that the bottom 3 mm of the bar is submerged in water; (3) letting the bar soak overnight (15 hours); (4) turn the bar over and grade qualitatively for the combined amount of smear, and characteristics of smear, depth of smear on a scale where 10 equals no smear, 8.0-9.5 equals low smear amount, 5.0-7.5 equals moderate smears similar to most marketed bars, and 4.5 or less equals very poor smear.
Good Commercial soap bars, have smears of about 5 and 6, respectively.

**THE EXAMPLES**

The example bars are made using the above Batch Process unless otherwise specified.

| EXAMPLES A. B, & C | | | |
|---|---|---|---|
| | A | B | C |
| Ingredients | Wt. % | Wt. % | Wt. % |
| Sodium Myristic Soap (C14) | 20.00 | 14.88 | 15.24 |
| Myristic Free Fatty Acid (C14) | | 0.09 | 0.09 |
| Sodium Lauric Soap (C12) | | 1.74 | 2.00 |
| Lauric Free Fatty Acid (C12) | | 0.01 | 0.01 |
| Coconut Soap | | 0.78 | 0.16 |
| Coco Betaine | 6.00 | | |
| Sodium Lauryl Sarcosinate | 8.00 | | |
| Stearalkonium Chloride | 3.00 | | |
| Perfume | 0.50 | 0.50 | 0.50 |
| Sodium Chloride | 3.00 | 2.50 | 2.50 |
| Petrolatum | 15.00 | 12.80 | 12.80 |
| Miscellaneous | 0.86 | 1.21 | 3.47 |
| Glycerine | 15.00 | 5.00 | 5.50 |
| Dimethicone | | 1.50 | 1.50 |
| Sodium Cocoyl Isethionate | | 24.44 | 5.00 |
| Sodium Isethionate | | 1.74 | 0.36 |
| Cocoamidopropyl Hydroxysultaine | | 2.00 | |
| Alkyl Glyceryl Ether Sulfonate | | | 19.00 |
| Mineral Oil | | 3.20 | 3.20 |
| Water | 28.64 | 27.61 | 28.67 |
| Hardness (mm) | 5.8 | 10.0 | 10.7 |
| Smear | 7.0 | 8.0 | 6.0 |
| Lather | 4.0 | 7.0 | 7.5 |
| Consistency | 1080-1345 | 354-430 | 354-430 |
| G' at 1 Hz. | 25M-40M | 8500-9300 | 8500-9300 |
| G" at 1 Hz. | 23.4M-36.4M | 6700-7000 | 6700-7000 |
| Overall User Acceptance Rating | 47 | 68 | 68 |
| * Mostly AGS by-products | | | |

Examples B and C are highly preferred examples. Example A has "overall user acceptance (100 is considered excellent and 0 poor)" much lower than Examples B and C. Examples B and C are lipid blends containing petrolatum, mineral oil and silicone. Example A contains only petrolatum. Example A also gives skin feel impressions of being "very sticky", "greasy", "tacky feeling".

| EXAMPLES D-G | | | | |
|---|---|---|---|---|
| EXAMPLES SHOWING USE OF DIFFERENT PREFERRED LIPIDS | | | | |
| | D | E | F | G |
| Ingredients | Wt. % | Wt. % | Wt. % | Wt. % |
| Sodium Myristic Soap | 20.00 | 20.00 | 20.00 | 20.00 |
| Coco Betaine | 6.00 | 6.00 | 6.00 | 6.00 |
| Sodium Lauryl Sarcosinate | 8.00 | 8.00 | 8.00 | 8.00 |
| Stearylkonium Chloride | 3.00 | 3.00 | 3.00 | 3.00 |
| Perfume | 0.50 | 0.50 | 0.50 | 0.50 |
| Sodium Chloride | 3.00 | 3.00 | 3.00 | 3.00 |
| Glycerine | 15.00 | 15.00 | 15.00 | 15.00 |
| Petrolatum | 12.00 | 13.65 | 12.00 | 15.00 |
| Mineral Oil | 3.00 | | 3.00 | |
| Cetyl Ricinoleate | | 3.00 | | |
| Dimethicone | | | 3.00 | |
| Isopropyl Palmitate | | | 0.50 | |
| Merquat 550 Polyquaternium 7 | | | 0.75 | |
| JR 400 Polyquaternium 10 | | | 0.75 | |
| Miscellaneous Minors | 0.86 | 0.86 | 0.60 | 0.86 |
| Water | 28.64 | 28.64 | 25.40 | 28.64 |
| Hardness) | - | | 6.8 | 8.9 |
| Smear | 8.0 | | 8.5 | 7.0 |
| Lather | 2.5 | | 3.0 | 4.0 |
| Consistency, k | 354-430 | | 354-430 | 1080-1345 |
| G' at 1Hz | 8500-9300 | | 8500-9300 | 25M-40M |
| G" at 1Hz | 6700-7000 | | 6700-7000 | 23.4M-36.4M |
| LDV (range) | 44.5 | | | 38-89 |

Examples D, E and F are preferred examples and use lipids blends selected from: Mineral oil, Isopropyl Palmitate, Cetyl Ricinoleate and Petrolatum. Example D is less sticky on the skin than Example G due to Example D's lower consistency k; even though they have comparable levels of Deposition.

| EXAMPLES H AND I | | |
|---|---|---|
| EXAMPLES SHOWING USE OF DIFFERENT PREFERRED LIPID BLENDS | | |
| | H | I |
| Ingredients | Wgt.% | Wgt. % |
| Sodium Myristic Soap (C14) | 14.38 | 14.38 |
| Coco Betaine | | |
| Coconut Soap | 0.62 | 0.62 |
| Sodium Lauryl Sarcosinate | | |
| Perfume- Odessa | 0.50 | 0.50 |
| Sodium Chloride | 2.80 | 2.80 |
| Petrolatum | 14.40 | 14.40 |
| Miscellaneous Minors | 2.21 | 2.21 |
| Glycerine | 5.00 | 5.00 |
| Sodium Cocoyl Isethionate | 28.00 | 28.00 |

(continued)

| EXAMPLES H AND I | | |
|---|---|---|
| EXAMPLES SHOWING USE OF DIFFERENT PREFERRED LIPID BLENDS | | |
| | H | I |
| Ingredients | Wgt.% | Wgt. % |
| Sodium Isethionate | 1.70 | 1.70 |
| Octyl Isononanoate | | 3.60 |
| Polydecene homopolymer | 3.60 | |
| Water | 26.79 | 26.79 |
| | | |
| Hardness (mm) | | |
| Smear | | |
| Lather | | |
| Consistency, k | 283-292 | |
| G' at 1Hz | 5881-7160 | |
| G" at 1Hz | 6118-6805 | |
| LDV (range) | | |

Examples H and I use lipids blends selected from: Octyl Isononanoate, Polydecene and Petrolatum. These preferred lipid blends generally increase or maintain the slipperiness of the rinse while reducing the coated feel of pure petrolatum (Example G).

| EXAMPLES J-M | | | | |
|---|---|---|---|---|
| Ingredient | J | K | L | M |
| Myristic fatty Acid | | | | |
| Sodium Myristic Soap | | | 20 | 30 |
| Sodium Palmitic Soap | | 19.9 | | |
| Palmitic Acid | | .1 | | |
| Sodium Lauric Soap | 19.9 | | | |
| Lauric Acid | .1 | | | |
| Coco Betaine | 6 | 6 | 6 | 5 |
| Sodium Lauryl Sarcosinate | 8 | 8 | 8 | 7 |
| Glycerine | 15 | 15 | 15 | 13 |
| NaCl | 3 | 3 | 3 | 3 |
| Perfume | 0.5 | 0.5 | 0.5 | 0.5 |
| Petrolatum | 15 | 15 | 15 | 14 |
| Miscillaneous. | 0.86 | .86 | 0.9 | 0.8 |
| Water | 28.64 | 28.64 | 29 | 24 |
| Stearalkonium Chloride | 3 | 3 | 3 | 3 |
| Lather | 1.5 | 0.5 | 2.5 | 3.5 |
| Smear | | | 9.0 | 8.5 |
| Hardness. | 6.47 | 9.23 | 6.4 | 4.8 |
| LDV | 136 | 12 | 42.5 | 4.5 |

Examples J, K and L are preferred bars. Different chain length soaps are used. Example J and L are more preferred than Example K, since Examples J and L have a soap fatty acid material with at least 25 % being C12 or C14. Examples L and M use of different levels of soap fatty acid material. All of these examples use a cationic surfactant: Stearalkonium Chloride, and Anionic Surfactants: Sodium Lauryl Sarcosinate and Coco Betaine

| EXAMPLES N-Q | | | | |
|---|---|---|---|---|
| LEVEL AND RATIO OF SOAP TO FATTY ACID MATERIAL | | | | |
| Series: Acid Soap | | | | |
| Ingredient | N | O | P | Q |
| Sodium Stearic Soap | 10.5 | 15.49 | 5.47 | 3.74 |
| Stearic Acid | 10.5 | 5.16 | .04 | 6.95 |
| Sodium MyristicSoap | 4.5 | | 6.2 | 1.78 |
| Myristic Acid | 4.5 | | .05 | 3.30 |
| Sodium Lauric Soap | | 6.75 | | 0.05 |
| Lauric Acid | | 2.25 | | 0.09 |
| Coconut Soap | | .26 | .73 | |
| Coconut Fatty Acid | | .09 | .01 | |
| Sodium Alkyl Isethionate. | | 16 | 23 | 21.60 |
| Sodium Isethionate. | | 0.95 | 1.63 | 1.22 |
| Coco Betaine | 2 | | | |
| Sodium Lauryl Sarcosinate | 10.5 | | | |
| Sodium Glycerylether Sulphonate | | | | 3.6 |
| Cocoamidopropyl/ HydroxySultaine | | | | 1.08 |
| Parrafin | | | | 3.6 |
| Sodium PalmiticSoap | | | | |
| Glycerine | 15 | 6 | 7 | 16.4 |
| NaCl | 3 | 2 | 2.5 | 0.5 |
| Perfume | 0.5 | 0.5 | .5 | .3 |
| Petrolatum | 15 | 16 | 12.8 | 15.0 |
| Mineral Oil | | | 3.2 | |
| Misc. | 0.81 | 1.48 | 1.14 | <2 |
| Water | 20.19 | 25.87 | 30.23 | 14.40 |
| Stearalkonium Chloride | 3 | 1.2 | | 2.60 |
| Ratio of Soap to fatty Acid | 1:1 | 3:1 | 125:1 | 1:2 |
| Level of Soap/Fatty Acid | 30.0 | 30.0 | 12.5 | 15.9 |
| Hardness. | | | FPB | FPB |
| LDV | 6.72 | 20.7 | 47.3 | 8.8 |
| Lipid Consistency | 1080-1345 | 1080-1345 | 354-430 | 354-430 |

Examples N, O, P and Q are preferred bars. Different soap to fatty acid ratios from 1:2 (Example Q) to 125: 1 (Example P) are used. The total level of soap fatty acid material ranges from 12.5 to 30 parts.

| EXAMPLES R-T | | | |
|---|---|---|---|
| LEVEL AND TYPE OF SYNTHETIC SURFACTANT | | | |
| Series: Water / NaCl | | | |
| Ingredient | R | S | T |
| Sodium MyristicSoap | 20 | 20 | 15.34 |
| Myristic Acid | | | .08 |
| Sodium Lauric Soap | | | 3.98 |
| Lauric Acid | | | .02 |
| Coconut Soap | | | .58 |
| Sodium Cocoyl Isethionate. | | | 18 |

(continued)

| EXAMPLES R-T | | | |
|---|---|---|---|
| LEVEL AND TYPE OF SYNTHETIC SURFACTANT | | | |
| Series: Water / NaCl | | | |
| Sodium Isethionate. | | | 1.28 |
| Coco Betaine | 6 | 6.3 | |
| Sodium Lauroyl Sarcosinate | 8 | 14.4 | |
| Glycerine | 8 | 10.5 | 5 |
| NaCl | 5 | 3 | 3 |
| Perfume | 0.5 | 0.5 | 0.5 |
| Petrolatum | 15 | 15 | 13.6 |
| Mineral Oil | | | 3.4 |
| Miscillaneous. | 0.86 | 1.27 | .89 |
| Water | 33.14 | 24.53 | 28.33 |
| Steamkonium Chloride | 3 | 4.5 | |
| Cocoamidopropyl/ Hydroxysultaine | | | 6.0 |
| Lather | 3 | 2 | |
| Smear | 6 | | |
| Hardness. | 9.67 | | 11.3 |
| Surfactant Level | 14.0 | 25.2 | 24.0 |

Examples R, S and T are preferred bars, that use different anionic synthetic surfactants: Sodium Lauryl Sarcosinate, Sodium Isethionate; different amphoteric surfactants: Coco Betaine, Cocoamidopropyl HydroxySultaine, and a cationic surfactant (deposition aid): Stearalkonium Chloride, The total level of synthetic surfactant ranges from 17.0 parts to 25.2.

| EXAMPLES U, V AND W | | | |
|---|---|---|---|
| LEVEL AND TYPE OF DEPOSITION AIDS: POLYOLS AND CATIONIC SURFACTANTS | | | |
| Preferred Bars | | | |
| Ingredient | U | V | W |
| Sodium Myristic Soap | 20 | 20 | 20 |
| Sodium Lauroyl Sarcosinate | 8 | 8 | 8 |
| Coco Betaine | 6 | 6 | 6 |
| Sodium Chloride | 3 | 3 | 3 |
| Glycerine | 15 | 15 | |
| Petrolatum | 15 | 15 | 15 |
| Perfume | .5 | .5 | .5 |
| Stearalkonium Chloride | 3 | | 3 |
| Micellaneous | .9 | .3 | .9 |
| Water | 29 | 32 | 44 |
| Hardness (mm) | 7.5 | 9.8 | 7.1 |
| Smear | 8.5 | 7.0 | 8.0 |
| Lather | 3.0 | 4.0 | 2.5 |
| LDV | 62 | 10 | 16 |

Examples U, V and W are preferred bars. Example U uses a highly preferred deposition aid polyol ingredient. Example U has 15 % Glycerine and deposits lipid at a significantly higher level than the non-polyol containing Example W. Example U also uses a second highly preferred deposition aid cationic surfactant ingredient. Example U has 3%

Stearalkonium Chloride and deposits lipid at a significantly higher level that the non-cationic surfactant containing Example V.

| EXAMPLE X AND Y | | |
|---|---|---|
| FORMULAE TESTED VIA CRYO SEM SHOWING LIPID IN WATER EMULSION | | |
| | X | Y |
| Ingredients | Wgt. % | Wet. % |
| Sodium Myristic Soap | 20.00 | 11.5 |
| Sodium Lauric Soap | | 2.9 |
| Sodium Coconut Soap | | 0.6 |
| Coco Betaine | 6.00 | |
| Sodium Lauryl Sarcosinate | 8.00 | |
| Sodium Cocoyl Isethionate | | 28.0 |
| Stearyl Dimethyl Benzyl Ammonium Chloride | 3.00 | |
| Perfume | 0.50 | 0.50 |
| Sodium Chloride | 3.00 | 3.00 |
| Sodium Isethionate | | 1.7 |
| Glycerine | 15.00 | 5.00 |
| Petrolatum | 15.00 | 14.40 |
| Mineral Oil | | 3.60 |
| Miscellaneous Minors | 0.86 | 2 |
| Water | 28.64 | 26.80 |
| Examples X and Y are preferred bars analyzed via Cryo SEM and are shown in the Figures. | | |

| EXAMPLES Z-CC | | | | |
|---|---|---|---|---|
| LEVEL AND TYPE OF THE HIGHLY PREFERRED OPTIONAL POLYOL | | | | |
| Series: Solvent | | | | |
| Ingredient | Z | AA | BB | CC |
| Sodium Myristic Soap | 17.91 | 17.2 | 15.89 | 15.89 |
| Myristic Acid | .09 | .09 | .09 | .09 |
| Sodium Lauric Soap | 1.99 | 1.99 | 1.84 | 1.84 |
| Lauric Acid | .01 | .01 | .01 | .01 |
| Coconut Soap | | .71 | .67 | .67 |
| Sodium Cocoyl Isethetionate. | | 22 | 21 | 21 |
| Sodium Isethionate. | | 1.56 | 1.56 | 1.56 |
| Sorbitol | 7 | 5.5 | | |
| Alkyl Glyceryl Ether/ Sulfonate | 20 | | | |
| Propylene Glycol | | | 3 | 6 |
| Glycerine | | | 5.1 | 2.1 |
| NaCl | 2.5 | 2 | 2.2 | 2.2 |
| Perfume | 0.5 | 0.5 | 0.5 | .05 |
| Petrolatum | 13.6 | 12.8 | 12.8 | 12.8 |
| Mineral Oil | 3.4 | 3.2 | 3.2 | 3.2 |
| Miscellaneous. | 4.03 | 1.6 | 1.04 | 1.04 |
| Water | 26.67 | 26.24 | 27 | 27 |
| Dimethicone | 1.5 | 1.5 | 1.5 | 1.5 |
| Merquatt 550 Polymer | 0.8 | 0.8 | 0.8 | 0.8 |

(continued)

| EXAMPLES Z-CC | | | | |
|---|---|---|---|---|
| LEVEL AND TYPE OF THE HIGHLY PREFERRED OPTIONAL POLYOL | | | | |
| Series: Solvent | | | | |
| Ingredient | Z | AA | BB | CC |
| Coco Hydroxy Sultaine | | 1.3 | 1.8 | 1.8 |
| Hardness. | 8.3 | 10.1 | | |
| LDV | 78 | 142 | 188 | 35 |

Examples Z, AA, BB and CC are highly preferred bars, and use different types of the highly preferred optional ingredients (deposition aid): propylene glycol, sorbitol and glycerine.

| EXAMPLES DD-FF | | | |
|---|---|---|---|
| LIPID LEVEL | | | |
| Ingredient | DD | EE | FF |
| Sodium MyristicSoap | 20 | 20 | 20 |
| Myristic Acid | | | |
| Coco Betaine | 6 | 6 | 6 |
| Sodium Lauryl Sarcosinate | 8 | 8 | 8 |
| Glycerine | 18.5 | 15 | 13.4 |
| NaCl | 3 | 3 | 3 |
| Perfume | 0.5 | 0.5 | 0.5 |
| Petrolatum | 10 | 15 | 20 |
| Mineral Oil | | | |
| Miscillaneous | .6 | .8 | .6 |
| Water | 31.9 | 28.7 | 27 |
| Dimethicone | | | |
| Stearalkonium Chloride | 1.5 | | 1.5 |
| Behenyl Trimethyl/ Ammonium Chloride | | 3.0 | |
| Lather | 3.5 | 3.0 | 3.5 |
| Smear | 8 | 75 | 7.5 |
| LDV | 1.99 | 41 | 43.2 |
| Lipid Consistency | 1080-1345 | 1080-1345 | 1080-1345 |

Examples EE and FF are preferred bars. Example DD has a lower deposition than preferred. These use different levels of lipid from 10 to 30 parts. Example EE contains 3.0 % of a highly preferred cationic surfactant (deposition aid): Behenyl Trimethyl Ammonium Chloride.

| EXAMPLES GG-JJ | | | | |
|---|---|---|---|---|
| LEVEL, TYPE AND RATIO OF ANIONIC SYNTHETIC SURFACTANTS | | | | |
| Ingredient | GG | HH | II | JJ |
| Sodium Myristic Soap | 20 | 20 | 20 | 20 |
| Sodium Lauroyl Sarcosinate | 8 | | 14 | 14 |
| Coco Betaine | | | | 6 |
| Alkyl glyceryl Sulfonate | 6 | | | |
| Sodium Cocoyl Isethionate | | 12.0 | | |
| Sodium Lauroyl Isethionate | | 2.0 | | |

(continued)

| EXAMPLES GG-JJ | | | | |
|---|---|---|---|---|
| LEVEL, TYPE AND RATIO OF ANIONIC SYNTHETIC SURFACTANTS | | | | |
| Ingredient | GG | HH | II | JJ |
| Sodium Chloride | 3 | 3 | 3 | 3 |
| Glycerine | 15 | 15 | 15 | 15 |
| Petrolatum | 15 | 15 | 15 | 15 |
| Perfume | 0.5 | 0.5 | 0.5 | 0.5 |
| Stearalkonium chloride | 3 | 3 | 3 | 3 |
| Micellaneous | <2 | <2 | <2 | <2 |
| Water | 28 | 28 | 28 | 23 |
| Hardness (mm) | 10.5 | 11.1 | 9.3 | 8.5 |
| Smear | 8.5 | 6.0 | 9.0 | 6.5 |
| Lather | 2.0 | 3.0 | 1.0 | 3.5 |
| LDV | 22 | 7 | 56 | 72 |

| EXAMPLES KK AND LL | | |
|---|---|---|
| GELLING AGENTS, USED TO MODIFY LIPID RHEOLOGY | | |
| Ingredient | KK | LL |
| Sodium MyristicSoap | 19.22 | 19.10 |
| Sodium Lauric Soap | .39. | |
| Na coconut Soap | .39 | |
| Sodium Lauroyl Sarcosinate | 12.00 | |
| Sodium cocooyl isethionate | 12.00 | 28.00 |
| Sodium Chloride | 2.00 | 2.80 |
| Glycerine | 10.00 | 5.00 |
| Petrolatum | 8.30 | |
| *Polydecene gel | 8.30 | |
| Polydecene | | 18.00 |
| Misc | < 2 | <2 |
| Water | 25.09 | 27.07 |
| LDV | 68 | 9 |

*Polydecene gel consists of 17.3 % sucrose polyester, 69.6 % polydecene, 12.9 % polyethylene polymer gellant.

Examples KK and LL are highly preferred bars, and highlight the use of different lipid types and specifically the use of oil thickening polymers, such as polyethylene to modify the rheological properties of the lipid.

| EXAMPLE MM | |
|---|---|
| COMPARATIVE EXAMPLE | |
| Ingredients | MM Wgt. % |
| Sodium Myristic Soap | 28.00 |
| Magnesium Myristic Soap | 5.00 |
| Myristic fatty Acid | 0.50 |
| Coco Betaine | 10.00 |
| Sodium Lauryl Sarcosinate | 3.00 |
| Perfume | 0.50 |
| Sodium Chloride | 2.58 |

(continued)

| EXAMPLE MM | |
|---|---|
| COMPARATIVE EXAMPLE | |
| Ingredients | MM Wgt. % |
| Propylene Glycol | 3.50 |
| Petrolatum | 22.50 |
| Miscellaneous Minors | 0.84 |
| Water | 24.44 |

Comparative Example MM is made by a freezer process as disclosed in US-A-5 340 492 to Kacher et al., filed 11/01/91,and US-A-5 425 892, Taneri et al., 03/24/93. It has a rigid crystalline structure, but deposits less than 3 µg of lipid per sq cm of skin tested using Lipid Depostion Protocol 1.

| **EXAMPLE NN** | |
|---|---|
| Ingredients | NN Wgt.% |
| Sodium Myristic Soap | 15.78 |
| Myristic Fatty Acid | 0.09 |
| Sodium Lauric Soap | 1,84 |
| Lauric Fatty Acid | 0.01 |
| Coconut Soap | 0.78 |
| Sodium Coco Isethionate | 24.44 |
| Sodium Isethionate | 1.74 |
| Cocoamidopropyl Hydroxysultaine | 2.00 |
| Perfume | 0.50 |
| Sodium Chloride | 2.50 |
| Glycerine | 5.00 |
| Petrolatum | 14.40 |
| Mineral Oil | 1.60 |
| Miscellaneous Minors | 1.21 |
| Water | 28.11 |
| Hardness | 9.50 |
| Smear | 5.5 |
| Lather | 7.5 |
| LDV | 50.5 |

Example NN is a highly preferred bar made by an In-Line Mixing Process as described above.

## Claims

**1.** A lathering skin cleansing bar composition comprising b*y weight parts of the bar composition:*

(a) from 5 parts to 40 parts of a lipid skin moisturizing agent, selected from the group of organic lipids, which are hydrophobic as defined by having a combined Vaughan Solubility Parameter (VSP) of from 5 to less than 10; and mixtures thereof;
(b) from 15 parts to 40 parts of a rigid semi-continuous, interlocking open mesh crystalline network structure consisting essentially of a fatty acid soap material which is a mixture of fatty acid soap and fatty acid, said fatty acid soap material having at least 75 % saturated alkyl chains; said alkyl chains being selected from chains of from 8 to 22 carbon atoms, and mixtures thereof; wherein said soap and said fatty acid are present in a ratio of from 2:1 to 10,000:1; and;
(c) from 1 part to 50 parts of a lathering synthetic surfactant; and
(d) from 10 parts to 50 parts water,

wherein said water and said lipid are predominantly within interstices of said open mesh crystalline network; wherein said bar composition has a Lipid Deposition Value of 3 to 1000..

2. The lathering skin cleansing bar composition of Claim 1 wherein at least 80 % of said fatty acid soap material has the following structure:

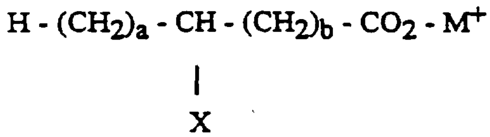

wherein:

a+b= 8 to 20
each a, b = 0 to 20
X = H, OR, O-CO-R, R, or mixtures thereof
R = C1-C3 alkyl, H, or mixtures thereof
M = Na, (½Mg), or mixtures thereof, and;

and wherein said fatty acid soap material has at least 85 % saturated alkyl chains; and wherein about 80 % to 100 % of said saturated alkyl chains are selected from the group consisting of 12 to 18 carbon atoms chains; and wherein said lipid is 10 to 40 parts b*y weight of the bar composition;* wherein said lipid has a viscosity consistency k value of 5 poise to 5,000 poise at 35 C; and wherein said lipid has a a shear index at 35°C in the range 0.1 to 0.8.

3. The lathering skin cleansing bar composition of Claim 2; wherein said lathering synthetic surfactant is from 5 to 40 parts b*y weight of the bar composition*; and wherein said lathering synthetic surfactant is selected from anionic surfactants; nonionic surfactants, amphoteric surfactants, and mixtures thereof; and wherein said lathering synthetic surfactant has a critical micelle concentration (CMC) equilibrium surface tension value of from 15 to 50 dynes per cm at 25°C; and said water is 15 to 45 parts; and wherein said lipid is from 10 to 35 parts b*y weight of the bar composition; and*, wherein said lipid is selected from hydrocarbon oils and waxes, silicone oils, di and tri-glyceride fats and oils, acetoglyceride esters, alkyl esters, alkenyl esters, polyol fatty acid polyesters, lanolin and its derivatives, wax esters, beeswax derivatives, vegetable waxes, sterols and phospholipids; and wherein said bar has a Lipid Deposition Value of 5 to 500;

**and** wherein;

said hydrocarbon oil and wax is **preferably** selected from petrolatum, mineral oil micro-crystalline waxes, polyalkenes, paraffin, cerasin, ozokerite, polyethylene and perhydrosqualene;

said silicone oil is **preferably** selected from : dimethicones, cyclomethicones, alkyl siloxanes, polymethylsiloxanes and methylphenylpolysiloxanes;

said di and tri glyceride is **preferably** selected from : hydroxylated milk glyceride, castor oil, soy bean oil, maleated soy bean oil, safflower oil, cotton seed oil, corn oil, walnut oil, peanut oil, olive oil, cod liver oil, almond oil, avocado oil, palm oil and sesame oil;

said alkyl ester is **preferably** selected from isopropyl esters of fatty acids and alkyl esters of riconoic acid isopropyl palmitate, isopropyl myristate, cetyl riconoleate and stearyl riconoleate; hexyl laurate, isohexyl laurate, myristyl myristate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, oleyl myristate, oleyl stearate and oleyl oleate. and cetyl lactate;

said lanolin based material is **preferably** selected from : lanolin oil, lanolin wax, lanolin alcohol, lanolin fatty acid, isopropyl lanolate, acetylated lanolin, acetylated lanolin alcohols, lanolin alcohol linoleate, lanolin alcohol riconoleate;

said wax esters is **preferably** selected from beeswax and beeswax derivatives, spermaceti, myristyl myristate, stearyl stearate;

said vegetable wax is **preferably** selected from carnauba and candelilla waxes;

said sterol is **preferably** selected from cholesterol, cholesterol fatty acid esters and homologs thereof;

said phospholipid is **preferably** selected from : lecithin and derivatives, Sphingo lipids, ceramides, glycosphingo lipids; and homologs thereof; and mixtures thereof;

and wherein said lipid is **preferably** from 10 parts to 30 parts, by weight of the bar composition; and wherein said bar has a Lipid Deposition Value in the range 10 to 500; and wherein **preferably** at least 70 % of said lipid phase is composed of lipids selected from : petrolatum, mineral oil micro-crystalline waxes, polyalkenes, paraffin, cerasin, ozokerite, polyethylene and perhydrosqualene; dimethicones, cyclomethicones, alkyl siloxanes, polymethylsiloxanes and methylphenylpolysiloxanes, hydroxylated milk glyceride, castor oil, soy bean oil, maleated soy bean oil, safflower oil, cotton seed oil, corn oil, walnut oil, peanut oil, olive oil, cod liver oil, almond oil, avocado oil, palm oil and sesame oil; lanolin, lanolin oil, lanolin wax, lanolin alcohol, lanolin fatty acid, isopropyl lanolate, acetylated lanolin, acetylated lanolin alcohols, lanolin alcohol linoleate, lanolin alcohol riconoleate; and mixtures thereof.

**4.** The lathering skin cleansing bar composition of Claim 2 wherein said anionic synthetic surfactant parts is from 3 to 35 parts, and wherein said lathering anionic synthetic surfactant is selected from : acyl isethionates, acyl sarcosinates, alkylglycerylether sulfonates, methylacyl taurates, paraffin sulfonates, linear alkyl benzene sulfonates, N-acyl glutamates, alkyl sulfosuccinates, alpha sulfo fatty acid esters, alkyl ether carboxylates, alkyl phosphate esters, ethoxylated alkyl phosphate esters, alkyl amine oxides, alpha olefin sulphates, the alkyl ether sulfates (with 1 to 12 ethoxy groups), and mixtures thereof, wherein said surfactants contain C8 to C22 alkyl chains and wherein the counterion is selected from : Na, K, $NH_4$, $N(CH_2CH_2OH)_3$.

**5.** The lathering skin cleansing bar composition of Claim 4 wherein the fatty acid material parts is from 15 parts to 35 parts; wherein at least 25 % of the fatty acid soap material have alkyl chains of 12 to 14 carbon atoms, wherein at least 95 % of said alkyl chains are saturated; and wherein said soap is at least four times said fatty acid; and wherein said fatty acid soap is selected from at least 75 % sodium soap; and wherein said lipid parts is from 12 parts to 24 parts by weight of the bar; wherein the said bar has aLipid Deposition Value of 25 to 500; and wherein said water parts is from 15 to 35 parts, by weight;
and wherein said anionic surfactant is **preferably** from 8 to 30 parts selected from acyl isethionate, acyl sarcosinates, alkyl sulfosuccinates, alkylglycerylether sulfonates, methylacyl taurates, alkyl ether sulfates, alkyl sulfates, alkyl phosphate esters and mixtures thereof, wherein said surfactants contain C8 to C18 alkyl chains; and wherein the counterion is Na; and wherein said amphoteric synthetic surfactant is **preferably** from 1 to 10 parts selected from : alkyl-ampho mono- and di- acetates, alkyl betaines, alkyl sultaines, alkyl amidopropyl betaines, alkyl amidopropyl hydroxysultaines, and mixtures thereof, wherein said amphoteric surfactant contain C12 to C22 alkyl chains; and wherein said lathering synthetic surfactant has a critical micelle concentration (CMC) equilibrium surface tension value of from 15 to 45 dynes per cm; and wherein said lathering synthetic surfactant parts is **preferably** from 10 to 35 parts, by weight; and is **preferably** selected from : anionic and/or amphoteric synthetic surfactant and wherein said anionic surfactant is more **preferably** selected from sodium lauryl and coco isethionate, sodium lauryl and coco sarcosinates, sodium C12-C16 sulfosuccinates, sodium C12-16 alkylglycerylether sulfonates, sodium lauryl and coco taurates; and wherein said amphoteric surfactant is preferably selected from : lauryl and coco betaines, lauryl and coco hydroxy sultaines, and mixtures thereof; and wherein ratio of said anionic to amphoteric is **preferably** from 1:1 to 30:1.

**6.** The lathering skin cleansing bar composition of Claim 5, wherein said synthetic surfactant parts comprises from 3 to 17 parts of a nonionic lathering synthetic surfactant selected from : alkyl glucose amides, alkyl glucose esters, polyoxyethylene amides, fatty alkane amides, alkyl amine oxides, alkyl polyglucosides, polyoxy ethylene alkyl phenols, polyoxyethylene esters of fatty acids, EO/PO block co-polymers such as polyoxamines and poloxamers, sorbitan esters and alcohol esters, and mixtures thereof.

**7.** A lathering skin cleansing bar composition according to any of the preceding claims comprising b*y weight parts of the bar composition:*

(a) from 5 parts to 40 parts of a lipid skin moisturizing agent, selected from the group of organic lipids, which are hydrophobic as defined by having a Vaughan Solubility Parameter (VSP) of from 5 to less than 10; and mixtures thereof:
(b) from 15 parts to 40 parts of a rigid semi-continuous, interlocking open mesh crystalline network structure consisting essentially of a fatty acid soap material which is a mixture of fatty acid soap and fatty acid, said fatty acid soap material having at least 75% saturated alkyl chains; said alkyl chains being selected from chains of from 8 to 22 carbon atoms, and mixtures thereof; wherein said soap and said fatty acid are present in a ratio of from 2:1 to 10,000:1; and
(c) from 1 part to 50 parts of a lathering synthetic surfactant; and
(d) from 10 parts to 50 parts water,
(e) from 0.5 to 6 parts of a cationic surfactant; and

wherein said water and said lipid are predominantly within interstices of said open mesh crystalline network; wherein said bar composition has a Lipid Deposition Value of 3 to 1000; and wherein said cationic surfactant improves the Lipid Deposition Value; and wherein said cationic synthetic surfactant is **preferably** selected from alkyltrimonium chloride and methyl sulphate, alkyl trimonium chloride and methyl sulphate, alkyl alkonium chloride and methyl sulphate, and di-alkyl dimonium chloride and methyl sulphate, and mixtures thereof, wherein said cationic surfactant **preferably** contain C12 to C24 carbon atoms per alkyl chain; and wherein said cationic surfactant is more **preferably** selected from stearalkonium chloride, stearyltrimonium chloride, Di-stearyl-dimonium chloride, and mixtures thereof.

**8.** A lathering skin cleansing bar composition according to any of the preceding claims comprising b*y weight parts of the bar composition:*

(a) from 5 parts to 40 parts of a lipid skin moisturizing agent, selected from the group of organic lipids, which are hydrophobic as defined by having a Vaughan Solubility Parameter (VSP) of from 5 to less than 10; and mixtures thereof;
(b) from 15 parts to 40 parts of a rigid semi-continuous, interlocking open mesh crystalline network structure consisting essentially of a fatty acid soap material which is a mixture of fatty acid soap and fatty acid, said fatty acid soap material having at least 75% saturated alkyl chains; said alkyl chains being selected from chains of from 8 to 22 carbon atoms, and mixtures thereof; wherein said soap and said fatty acid are present in a ratio of from 2:1 to 10,000:1;
(c) from 1 part to 50 parts of a lathering synthetic surfactant; and
(d) from 10 parts to 50 parts water;
(e) from 0.5 to 35 parts water soluble, organic material is selected from a polyol of the structure:

$$R1 - O(CH_2 - CR2HO)_nH$$

where R1 = H, C1-C4 alkyl; R2 = H, $CH_3$ and n = 1 - 200; C2-C10 alkane diols; guanidine; glycolic acid and glycolate salts (e.g. ammonium and quaternary alkyl ammonium); lactic acid and lactate salts (e.g., ammonium and quaternary alkyl ammonium); polyhydroxy alcohols such as sorbitol, glycerol, hexanetriol, propylene glycol, hexylene glycol and the like; polyethylene glycol; sugars and starches; sugar and starch derivatives (e.g. alkoxylated glucose); panthenol (including D-, L-, and the D,L- forms); pyrrolidone carboxylic acid; hyaluronic acid; lactamide monoethanolamine; acetamide monoethanolamine; urea; and ethanol amines of the general structure $(HOCH_2CH_2)_xNH_y$ where x = 1-3; y = 0-2, and x+y = 3, and mixtures thereof; wherein said bar contains a non-volatile, organic material having a solubility of a least 50 % in water; and wherein said water soluble organic material at least 50 % soluble in water, and wherein said water and said lipid are predominantly within interstices of said open mesh crystalline network; wherein said bar composition has a Lipid Deposition Value of 3 to 1000.

**9.** The lathering skin cleansing bar composition of Claim 8 wherein said water soluble organic material parts is from 3 to 20 parts, by weight said bar; and wherein said organic material is selected from glycerine, polyoxypropylene (1) glycerol and polyoxypropylene (3) glycerol, sorbitol, butylene glycol, propylene glycol, sucrose, and urea and triethanolamine; and mixtures thereof; and wherein said lipid is from 15 parts to 25 parts, by weight of the bar; and wherein said synthetic surfactant is from 15 to 30 parts by weight of the bar; said soap is present at a level from 15 parts to 25 parts and said soap is essentially C-12-18 carboxylic acids, with at least 50 % being C12 or C14; and wherein at least 75 % of said lipid by weight of said lipid is selected from petrolatum, micro crystalline wax,

mineral oil or polydecene and mixes thereof; and wherein 0 to 25 % of said lipid by weight of said lipid is selected from dimethicone or alkyl polysiloxane and mixtures thereof; and wherein said water is **preferably** present at a level from 15 parts to 35 parts; and wherein said lipid parts is **preferably** from 15 to 25 parts, by weight of the composition at least 75 % of said lipid is selected from the petrolatum, micro crystalline wax, mineral oil or polydecene; and wherein said lipid has an elastic modulus (G') measured at 1 Hz. and 35°C in the range 1,000 to 80,000 dynes/sq. cm and has a viscous modulus (G") measured at 1 Hz. and 35°C in the range 500 to 300,000 dynes/ sq. cm.; and wherein said bar contains from 0.1 parts to 10 parts of a polymeric, nonionic, cationic or hydrophobically modified polymeric skin feel aid, selected from cationic polysaccharides of the cationic guar gum class with molecular weights of 1,000 to 3,000,000, cationic and nonionic homopolymers derived from acrylic and/ or methacrylic acid, cationic and nonionic cellulose resins; cationic copolymers of dimethyldialkylammonium chloride and acrylic acid; cationic homopolymers of dimethyldialkylammonium chloride; cationic polyalkylene and ethoxypolyalkylene imines; and mixes thereof; and wherein said polymeric, nonionic or cationic polymeric skin feel aid parts is **preferably** from 0.25 parts to 3 parts, by weight, and is **preferably** selected from hydroxypropyl guar, guar hydroxypropyltrimonium chloride, polyquaternary 3, 5, 6, 7, 10, 11 and 24 and mixes thereof.

**10.** A lathering skin cleansing bar composition according to any of the preceding claims comprising *by weight parts of the bar composition*:

(a) from 5 parts to 40 parts of a lipid skin moisturizing agent, selected from the group of organic lipids, which are hydrophobic as defined by having a Vaughan Solubility Parameter (VSP) of from 5 to less than 10; and mixtures thereof;
(b) from 15 parts to 40 parts of a rigid semi-continuous, interlocking open mesh crystalline network structure consisting essentially of a fatty acid soap material which is a mixture of fatty acid soap and fatty acid, said fatty acid soap material having at least 75 % saturated alkyl chains; said alkyl chains being selected from chains of from 8 to 22 carbon atoms, and mixtures thereof ; wherein said soap and said fatty acid are present in a ratio of from 2:1 to 10,000:1;
(c) from 1 part to 50 parts of a lathering synthetic surfactant; and
(d) from 10 parts to 50 parts water,

wherein said water and said lipid are predominantly within interstices of said open mesh crystalline network; wherein said bar composition has a Lipid Deposition Value of 3 to 1000, and . wherein said bar contains from 0.1 parts to 50 parts of other ingredients selected from:

from 0.5 to 5 parts potassium soap;
from 0.5 to 5 parts alkoxylated ammonium and/or alkylammonium and/or alkoxylated aliphatic amines and/or polyethoxylated amine soap;
from 0.5 to 15 parts calcium soap;
from 0.5 to 50 parts of impalpable water insoluble materials selected from calcium carbonate and talc;
from 0.5 to 25 parts of aluminosilicate clay and/or other clays; wherein said aluminosilicates and clays are selected from zeolites, kaolin, kaolinite, bentonite, halloysite, calcined clays, montmorillonite, illite and attapulgite.
from 0.5 to 25 parts dextrin, corn and wheat starch;
from 0.1 to 15 parts of salt and salt hydrates; and mixtures thereof; and wherein said salt and salt hydrate have a cation selected from sodium, potassium, magnesium, calcium, aluminum, lithium ammonium, alkoxylated ammonium, alkylammonium, alkoxylated aliphatic amines, polyethoxylated amines; and wherein said salt and/or salt hydrate have an anion selected from chloride, sulfate, isethionate, bromide, metasilicate, orthophosphate, pyrophosphate, polyphosphate, metaborate, tetraborate, carbonate, bicarbonate, hydrogen phosphate, methyl sulfate, and mono- and polycarboxylate of 1 to 6 carbon atoms or less;
from 0.1 to 1 parts whitening aid;
from 0.1 to 2 parts of a fragrance or perfume;
from 0.1 to 5 parts of a chelant, preserve or anti fungal/anti microbial/bacterial agent.

**11.** A lathering skin cleansing bar composition according to any of the preceding claims comprising *by weight parts of the bar composition:*

(a) from 5 parts to 40 parts of a lipid skin moisturizing agent, selected from the group of organic lipids, which are hydrophobic as defined by having a Vaughan Solubility Parameter (VSP) of from 5 to less than 10; and mixtures thereof;

(b) from 15 parts to 40 parts of a rigid semi-continuous, interlocking open mesh crystalline network structure consisting essentially of mixtures of mono and di-carboxylic fatty acid soap material selected from mixtures of mono and di-carboxylic fatty acid soap and and mono and di carboxylic fatty acid; wherein said fatty acid soap material has at least 75 % saturated alkyl chains; said alkyl chains being selected from the chains of from 8 to 22 carbon atoms, and mixtures thereof; and wherein said soap and said fatty acid have a ratio of from 2:1 to 10,000:1;
(c) from 1 part to 50 parts of a lathering synthetic surfactant; and
(d) from 10 parts to 50 parts water;

wherein said water and said lipid are predominantly within interstices of said open mesh crystalline network; wherein said bar composition has a Lipid Deposition Value of at least 3 to 1000.

**12.** The lathering skin cleansing bar composition of Claim 1, wherein said lipid has a a shear index at 35°C in the range 0.1 to 0.5 and a consistency k at 35°C in the range 10 to 3,000 poise; and wherein said lipid preferably has an elastic modulus (G') measured at 1Hz and 35°C in the range 25 to 100,000 dynes/sq. cm and has an viscous modulus (G") measured at 1Hz and 35 C in the range 25 to 500,000 dynes/ sq. cm.; and wherein said elastic modulus (G') is more preferably in the range of 5,000 to 50,000 dynes/sq. cm and has a viscous modulus (G") in the range of 5,000 to 100,000 dynes/ sq. cm.

**13.** The lathering skin cleansing bar composition of Claim 2, wherein said lipid is from 12 parts to 25 parts, by weight of the bar composition; and wherein said lipid has a k value of 50 to 2000 poise; and wherein at least 70 % of said lipid is composed of lipids selected from petrolatum, mineral oil, micro crystalline wax, paraffin, polyethylene, polybutene, polydecene, dimethicones, alkyl siloxane, cyclomethicones, lanolin, lanolin oil, lanolin wax; with the remainder selected from: isopropyl palmitate, cetyl riconoleate, octyl isononanoate, octyl palmitate, isocetyl stearate, hydroxylated milk glyceride; and mixtures thereof; and wherein said water forms an aqueous phase, wherein, 20 to 95 % of said aqueous phase is water; and wherein the ratio of said water to said lipid parts is preferably greater than 1:1; and wherein said water is 15 to 35 parts; and wherein said aqueous phase and said lipid form a lipid in water emulsion entrapped in said interlocking open mesh crystalline network; and wherein said bar produces an unstable lipid in water emulsion when subjected to Lipid Stability Centrifuge Test.

## Patentansprüche

**1.** Schäumende Hautreinigungszusammensetzung in Stückform, umfassend bezogen auf Gewichtsteile der Stückformzusammensetzung:

(a) 5 Teile bis 40 Teile eines Lipid-Hautfeuchthaltemittels, gewählt aus der Gruppe organischer Lipide, welche hydrophob sind, wie dadurch definiert, dass sie einen kombinierten Vaughan-Solubilitäts-Parameter (VSP) von 5 bis weniger als 10 aufweisen; und Mischungen hiervon;
(b) 15 Teile bis 40 Teile einer starren, semikontinuierlichen, ineinandergreifenden, offenmaschigen, kristallinen Netzwerkstruktur, bestehend im wesentlichen aus einem Fettsäureseifenmaterial, das eine Mischung aus Fettsäureseife und Fettsäure ist, wobei das Fettsäureseifenmaterial mindestens 75% gesättigte Alkylketten aufweist; die Alkylketten gewählt sind aus Ketten mit 8 bis 22 Kohlenstoffatomen, und Mischungen hiervon; wobei die Seife und die Fettsäure in einem Verhältnis von 2:1 bis 10.000:1 vorliegen; und
(c) 1 Teil bis 50 Teile eines schäumenden, synthetischen Tensids; und
(d) 10 Teile bis 50 Teile Wasser;

wobei das Wasser und das Lipid sich vorwiegend innerhalb Zwischenräumen des offenmaschigen, kristallinen Netzwerks befinden; wobei die Stückformzusammensetzung einen Lipid-Depositions-Wert von 3 bis 1.000 aufweist.

**2.** Schäumende Hautreinigungszusammensetzung in Stückform nach Anspruch 1, wobei mindestens 80% des Fettsäureseifenmaterials die folgende Struktur aufweist:

$$H - (CH_2)_a - CH - (CH_2)_b - CO_2 - M^+$$
$$\quad\quad\quad\quad | $$
$$\quad\quad\quad\quad X$$

worin:

a + b = 8 bis 20,
jedes a, b = 0 bis 20,
X = H, OR, O-CO-R, R, oder Mischungen hiervon,
R = $C_1$-$C_3$-Alkyl, H, oder Mischungen hiervon,
M = Na, (1/2Mg) oder Mischungen hiervon, und

wobei das Fettsäureseifenmaterial mindestens 85% gesättigte Alkylketten aufweist; und wobei etwa 80% bis 100% der gesättigten Alkylketten aus der Gruppe gewählt sind, bestehend aus Ketten mit 12 bis 18 Kohlenstoffatomen; und wobei das Lipid 10 bis 40 Gewichtsteile der Stückformzusammensetzung ausmacht; wobei das Lipid einen Viskositätskonsistenz-k-Wert von 5 poise bis 5.000 poise bei 35°C aufweist; und wobei das Lipid einen Scherindex bei 35°C im Bereich von 0,1 bis 0,8 aufweist.

**3.** Schäumende Hautreinigungszusammensetzung in Stückform nach Anspruch 2, wobei das schäumende synthetische Tensid 5 bis 40 Gewichtsteile der Stückformzusammensetzung ausmacht; und wobei das schäumende Tensid gewählt ist aus anionischen Tensiden; nichtionischen Tensiden, amphoteren Tensiden und Mischungen hiervon; und wobei das schäumende synthetische Tensid einen kritische Micellenkonzentration(CMC)-Gleichgewichts-Oberflächenspannungswert von 15 bis 50 dynes per cm bei 25°C aufweist; und das Wasser 15 bis 45 Teile ausmacht; und wobei das Lipid 10 bis 35 Gewichtsteile der Stückformzusammensetzung ausmacht; und wobei das Lipid gewählt ist aus Kohlenwasserstoffölen und Wachsen, Siliconölen, Di- und Triglyceridfetten und -ölen, Acetoglyceridestern, Alkylestern, Alkenylestern, Polyolfettsäurepolyestern, Lanolin und dessen Derviaten, Wachsestern, Bienenwachsderivaten, pflanzlichen Wachsen, Sterolen und Phospholipiden; und wobei die Stückform einen Lipid-Depositions-Wert von 5 bis 500 aufweist;

und wobei

das Kohlenwasserstofföl und Wachs vorzugsweise gewählt ist aus Petrolatum, Mineralöl, mikrokristallinen Wachsen, Polyalkenen, Paraffin, Cerasin, Ozokerit, Polyethylen und Perhydrosqualen;
das Siliconöl vorzugsweise gewählt ist aus Dimethiconen, Cyclomethiconen, Alkylsiloxanen, Polymethylsiloxanen und Methylphenylpolysiloxanen;
das Di- und Triglycerid vorzugsweise gewählt ist aus hydroxyliertem Milchglycerid, Rhizinusöl, Sojabohnenöl, maleiertem Sojabohnenöl, Safloröl, Baumwollsamenöl, Maisöl, Walnußöl, Erdnußöl, Olivenöl, Lebertran, Mandelöl, Avocadoöl, Palmöl und Sesamöl;
der Alkylester vorzugsweise gewählt ist aus Isopropylestern von Fettsäuren und Alkylestern von Ricinolsäure, Isopropylpalmitat, Isopropylmyristat, Cetylriconoleat und Stearylriconoleat; Hexyllaurat, Isohexyllaurat, Myristylmyristat, Isohexylpalmitat, Decyloleat, Isodecyloleat, Hexadecylstearat, Decylstearat, Isopropylisostearat, Diisopropyladipat, Diisohexyladipat, Dihexyldecyladipat, Diisopropylsebacat, Acylisononanoat, Laurylllactat, Myristyllactat, Oleylmyristat, Oleylstearat und Oleyloleat und Cetyllactat;
das Material auf Lanolinbasis vorzugsweise gewählt ist aus Lanolinöl, Lanolinwachs, Lanolinalkohol, Lanolinfettsäure, Isopropyllanolat, acetyliertem Lanolin, acetylierten Lanolinalkoholen, Lanolinalkohollinoleat, Lanolinalkoholriconoleat;
der Wachsester vorzugsweise gewählt ist aus Bienenwachs und Bienenwachsderivaten, Spermaceti, Myristylmyristat, Stearylstearat; das pflanzliche Wachs vorzugsweise gewählt ist aus Carnauba- und Candelilla-Wachsen;
das Sterol vorzugsweise gewählt ist aus Cholesterol, Cholesterolfettsäureestern und Homologen hiervon;
das Phospholipid vorzugsweise gewählt ist aus Lecithin und Derivaten, Sphingo-Lipiden, Ceramiden. Glykosphingolipiden; und Homologen hiervon; und Mischungen hiervon:

und wobei das Lipid vorzugsweise 10 bis 30 Gewichtsteile der Stückformzusammensetzung ausmacht; und wobei die Stückform einen Lipid-Depositions-Wert im Bereich von 10 bis 500 aufweist; und wobei vorzugsweise mindestens 70% der Lipidphase aus Lipiden zusammengesetzt ist, gewählt aus Petrolatum, Mineralöl,

mikrokristallinen Wachsen, Polyalkenen, Paraffin, Cerasin, Ozokerit, Polyethylen und Perhydrosqualen; Dimethiconen, Cyclomethiconen, Alkylsiloxanen, Polymethylsiloxanen und Methylphenylpolysiloxanen, hydroxyliertem Milchglycerid, Rhizinusöl, Sojabohnenöl, maleiertem Sojabohnenöl, Safloröl, Baumwollsamenöl, Maisöl, Walnußöl, Erdnußöl, Olivenöl, Lebertran, Mandelöl, Avocadoöl, Palmöl und Sesamöl; Lanolin, Lanolinöl, Lanolinwachs, Lanolinalkohol, Lanolinfettsäure, Isopropyllanolat, acetyliertem Lanolin, acetylierten Lanolinalkoholen, Lanolinalkohollinoleat, Lanolinalkoholriconoleat; und Mischungen hiervon.

**4.** Schäumende Hautreinigungszusammensetzung in Stückform nach Anspruch 2, wobei das anionische synthetische Tensid 3 bis 35 Teile ausmacht, und wobei das schäumende, anionische synthetische Tensid gewählt ist aus Acylisethionaten, Acylsarcosinaten, Alkylglycerylethersulfonaten, Methylacyltauraten, Paraffinsulfonaten, linearen Alkylbenzolsulfonaten, N-Acylglutamaten, Alkylsulfosuccinaten, alpha-Sulfofettsäureestern, Alkylethercarboxylaten, Alkylphosphatestern, ethoxylierten Alkylphosphatestern, Alkylaminoxiden, alpha-Olefinsulfaten, den Alkylethersulfaten (mit 1 bis 12 Ethoxygruppen), und Mischungen hiervon, wobei die Tenside $C_8$-$C_{22}$-Alkylketten enthalten und wobei das Gegenion gewählt ist aus Na, K, $NH_4$, $N(CH_2CH_2OH)_3$.

**5.** Schäumende Hautreinigungszusammensetzung in Stückform nach Anspruch 4, wobei das Fettsäurematerial 15 bis 35 Teile ausmacht; wobei mindestens 25% des Fettsäureseifenmaterials Alkylketten mit 12 bis 14 Kohlenstoffatomen aufweisen, wobei mindestens 95% der Alkylketten gesättigt sind; und wobei die Seife mindestens das Vierfache der Fettsäure beträgt; und wobei die Fettsäureseife gewählt ist aus mindestens 75% Natriumseife; und wobei das Lipid 12 bis 24 Gewichtsteile der Stückform ausmacht; wobei die Stückform einen Lipid-Depositions-Wert von 25 bis 500 aufweist; und wobei das Wasser 15 bis 35 Gewichtsteile ausmacht; und wobei das anionische Tensid vorzugsweise 8 bis 30 Teile ausmacht und gewählt ist aus Acylisethionaten, Acylsarcosinaten, Alkylsulfosuccinaten, Alkylglycerylethersulfonaten, Methylacyltauraten, Alkylethersulfaten, Alkylsulfaten, Alkylphosphatestern und Mischungen hiervon, wobei die Tenside $C_8$-$C_{18}$-Alkylketten enthalten; und wobei das Gegenion Na ist; und wobei das amphotere synthetische Tensid vorzugsweise 1 bis 10 Teile ausmacht und gewählt ist aus Alkyl-Amphomono- und -diacetaten, Alkylbetainen, Alkylsultainen, Alkylamidopropylbetainen, Alkylamidopropylhydroxysultainen und Mischungen hiervon, wobei das amphotere Tensid $C_{12}$-$C_{22}$-Alkylketten enthält; und wobei das schäumende synthetische Tensid einen kritische Micellenkonzentration(CMC)-Gleichgewichts-Oberflächenspannungswert von 15 bis 45 dynes pro cm aufweist; und wobei das schäumende synthetische Tensid vorzugsweise 10 bis 35 Gewichtsteile ausmacht und vorzugsweise gewählt ist aus anionischem und/oder amphoterem synthetischen Tensid, und wobei das anionische Tensid weiter vorzugsweise gewählt ist aus Natriumlauryl- und Cocoisethionat, Natriumlauryl- und Cocosarcosinaten, Natrium-$C_{12}$-$C_{16}$-sulfosuccinaten, Natrium-$C_{12-16}$-alkylglycerylethersulfonaten, Natriumlauryl- und Cocotauraten; und wobei das amphotere Tensid vorzugsweise gewählt ist aus Lauryl- und Cocobetainen, Lauryl- und Cocohydroxysultainen und Mischungen hiervon; und wobei das Verhältnis von anionisch zu amphoter vorzugsweise 1:1 bis 30:1 beträgt.

**6.** Schäumende Hautreinigungszusammensetzung in Stückform nach Anspruch 5, wobei das synthetische Tensid 3 bis 17 Teile eines nichtionischen, schäumenden, synthetischen Tensids umfaßt, gewählt aus Alkylglucoseamiden, Alkylglucoseestern, Polyoxyethylenamiden, Fettalkanamiden, Alkylaminoxiden, Alkylpolyglucosiden, Polyoxyethylenalkylphenolen, Polyoxyethylenestern von Fettsäuren, EO/PO-Blockcopolymeren, wie Polyoxyaminen und Polyoxameren, Sorbitanestern und Alkoholester, und Mischungen hiervon.

**7.** Schäumende Hautreinigungszusammensetzung in Stückform nach mindestens einem der vorangehenden Ansprüche, umfassend, bezogen auf Gewichtsteile der Stückformzusammensetzung:

(a) 5 bis 40 Teile eines Lipid-Hautfeuchthaltemittels, gewählt aus der Gruppe organischer Lipide, welche hydrophob sind, wie dadurch definiert, dass sie einen Vaughan-Solubilitäts-Parameter (VSP) von 5 bis weniger als 10 aufweisen; und Mischungen hiervon;
(b) 15 Teile bis 40 Teile einer starren, semikontinuierlichen, ineinandergreifenden, offenmaschigen, kristallinen Netzwerkstruktur, bestehend im wesentlichen aus einem Fettsäureseifenmaterial, das eine Mischung aus Fettsäureseife und Fettsäure ist, wobei das Fettsäureseifenmaterial mindestens 75% gesättigte Alkylketten aufweist; die Alkylketten gewählt sind aus Ketten mit 8 bis 22 Kohlenstoffatomen, und Mischungen hiervon; wobei die Seife und die Fettsäure in einem Verhältnis von 2:1 bis 10.000:1 vorliegen; und
(c) 1 Teil bis 50 Teile eines schäumenden, synthetischen Tensids; und
(d) 10 Teile bis 50 Teile Wasser;
(e) 0,5 bis 6 Teile eines kationischen Tensids; und

wobei das Wasser und das Lipid sich vorwiegend innerhalb Zwischenräumen des offenmaschigen, kristallinen

Netzwerks befinden; wobei die Stückformzusammensetzung einen Lipid-Depositions-Wert von 3 bis 1000 aufweist; und wobei das kationische Tensid den Lipid-Depositions-Wert verbessert; und wobei das kationische synthetische Tensid vorzugsweise gewählt ist aus Alkyltrimoniumchlorid und Methylsulfat, Alkyltrimoniumchlorid und Methylsulfat, Alkylalkoniumchlorid und Methylsulfat, und Dialkyldimoniumchlorid und Methylsulfat, und Mischungen hiervon, wobei das kationische Tensid vorzugsweise $C_{12}$-$C_{14}$-Kohlenstoffatome pro Alkylkette enthält; und wobei das kationische Tensid weiter vorzugsweise gewählt ist aus Stearalkoniumchlorid, Stearyltrimoniumchlorid, Di-Stearyldimoniumchlorid und Mischungen hiervon.

**8.** Schäumende Hautreinigungszusammensetzung in Stückform nach mindestens einem der vorangehenden Ansprüche, umfassend, bezogen auf Gewichtsteile der Stückformzusammensetzung:

(a) 5 bis 40 Teile eines Lipid-Hautfeuchthaltemittels, gewählt aus der Gruppe organischer Lipide, welche hydrophob sind, wie dadurch definiert, dass sie einen Vaughan-Solubilitäts-Parameter (VSP) von 5 bis weniger als 10 aufweisen; und Mischungen hiervon;
(b) 15 Teile bis 40 Teile einer starren, semikontinuierlichen, ineinandergreifenden, offenmaschigen, kristallinen Netzwerkstruktur, bestehend im wesentlichen aus einem Fettsäureseifenmaterial, das eine Mischung aus Fettsäureseife und Fettsäure ist, wobei das Fettsäureseifenmaterial mindestens 75% gesättigte Alkylketten aufweist; die Alkylketten gewählt sind aus Ketten mit 8 bis 22 Kohlenstoffatomen, und Mischungen hiervon; wobei die Seife und die Fettsäure in einem Verhältnis von 2:1 bis 10.000:1 vorliegen;
(c) 1 Teil bis 50 Teile eines schäumenden, synthetischen Tensids; und
(d) 10 Teile bis 50 Teile Wasser;
(e) 0,5 bis 35 Teile wasserlösliches, organisches Material, gewählt aus einem Polyol der Struktur:

$$R1 - O(CH_2 - CR2HO)_nH$$

worin R1 = H, $C_1$-$C_4$-Alkyl; R2 = H, $CH_3$, und n = 1-200; $C_2$-$C_{10}$-Alkandiolen, Guanidin; Glykolsäure und Glykolatsalzen (z.B. Ammonium und quaternäres Alkylammonium); Milchsäure und Lactatsalzen (z.B. Ammonium und quaternäres Alkylammonium); Polyhydroxyalkoholen, wie Sorbitol, Glycerin, Hexantriol, Propylenglykol, Hexylenglykol und dergleichen; Polyethylenglykol; Zuckern und Stärken; Zucker- und Stärkederivaten (z.B. alkoxylierte Glucose); Panthenol (einschließlich D-, L- und D,L-Formen); Pyrrolidoncarbonsäure; Hyaluronsäure; Lactamidmonoethanolamin; Acetamidmonoethanolamin; Harnstoff; und Ethanolaminen der allgemeinen Struktur $(HOCH_2CH_2)_xNH_y$, worin x = 1-3; y = 0-2, und x+y = 3, und Mischungen hiervon, wobei die Stückform ein nichtflüchtiges, organisches Material mit einer Löslichkeit von mindestens 50% in Wasser enthält; und wobei das wasserlösliche organische Material zu mindestens 50% wasserlöslich ist; und wobei sich das Wasser und das Lipid vorwiegend innerhalb Zwischenräumen des offenmaschigen, kristallinen Netzwerks befinden; wobei die Stückformzusammensetzung einen Lipid-Depositions-Wert von 3 bis 1000 aufweist.

**9.** Schäumende Hautreinigungszusammensetzung in Stückform nach Anspruch 8, wobei das wasserlösliche, organische Material 3 bis 20 Gewichtsteile der Stückform ausmacht; und wobei das organische Material gewählt ist aus Glycerin, Polyoxypropylen(1)-Glycerin und Polyoxypropylen(3)-Glycerin, Sorbitol, Butylenglykol, Propylenglykol, Sucrose, und Harnstoff und Triethanolamin; und Mischungen hiervon; und wobei das Lipid 15 bis 25 Gewichtsteile der Stückform ausmacht; und wobei das synthetische Tensid 15 bis 30 Gewichtsteile der Stückform ausmacht; die Seife in einem Anteil von 15 Teilen bis 25 Teilen vorliegt und die Seife im wesentlichen $C_{12-18}$-Carbonsäuren umfaßt, wobei mindestens 50% C12 oder C14 sind; und wobei mindestens 75% des Lipids, bezogen auf das Gewicht des Lipids, gewählt sind aus Petrolatum, mikrokristallinem Wachs, Mineralöl oder Polydecen und Mischungen hiervon; und wobei 0 bis 25% des Lipids, bezogen auf das Gewicht des Lipids, gewählt sind aus Dimethicon oder Alkylpolysiloxan und Mischungen hiervon; und wobei das Wasser vorzugsweise in einem Anteil von 15 Teilen bis 35 Teilen vorliegt; und wobei das Lipid vorzugsweise 15 bis 25 Teile des Gewichts der Zusammensetzung ausmacht, wobei mindestens 75% des Lipids gewählt sind aus Petrolatum, mikrokristallinem Wachs, Mineralöl oder Polydecen; und wobei das Lipid einen bei 1 Hz und 35°C gemessenen Elastizitätsmodul (G') im Bereich von 1.000 bis 80.000 $dynes/cm^2$ und einen bei 1 Hz und 35°C gemessenen Viskositätsmodul (G") im Bereich von 500 bis 300.000 $dynes/cm^2$ aufweist; und wobei die Stückform 0,1 bis 10 Teile einer polymeren, nichtionischen, kationischen oder hydrophob modifizierten, polymeren Hautanfühlhilfe umfaßt, gewählt aus kationischen Polysacchariden der kationischen Guargummi-Klasse mit Molekulargewichten von 1.000 bis 3.000.000, kationischen und nichtionischen Homopolymeren, abgeleitet aus Acryl- und/oder Methacrylsäure, kationischen und nichtionischen Celluloseharzen; kationischen Copolymeren von Dimethyldialkylammoniumchlorid und Acrylsäure; kationischen Homopolymeren von Dimethyldialkylammoniumchlorid; kationischen Polyalkylen- und Ethoxypolyalkyleniminen; und Mischungen hiervon, und wobei die polymere, nichionische oder kationische polymere Hautanfühlhilfe vorzugsweise 0,25 bis 3 Gewichtsteile ausmacht und vorzugsweise gewählt ist aus Hydroxypropylguar, Guarhydroxypropyltrimoniumchlorid, Polyquatanium 3, 5, 6, 7, 10, 11 und 24 und Mischungen hiervon.

**10.** Schäumende Hautreinigungszusammensetzung in Stückform nach mindestens einem der vorangehenden Ansprüche, umfassend, bezogen auf Gewichtsteile der Stückformzusammensetzung:

(a) 5 Teile bis 40 Teile eines Lipid-Hautfeuchthaltemittels, gewählt aus der Gruppe organischer Lipide, welche hydrophob sind, wie dadurch definiert, dass sie einen Vaughan-Solubilitäts-Parameter (VSP) von 5 bis weniger als 10 aufweisen; und Mischungen hiervon;
(b) 15 Teile bis 40 Teile einer starren, semikontinuierlichen, ineinandergreifenden. offenmaschigen, kristallinen Netzwerkstruktur, bestehend im wesentlichen aus einem Fettsäureseifenmaterial, das eine Mischung aus Fettsäureseife und Fettsäure ist, wobei das Fettsäureseifenmaterial mindestens 75% gesättigte Alkylketten aufweist; die Alkylketten gewählt sind aus Ketten mit 8 bis 22 Kohlenstoffatomen, und Mischungen hiervon; wobei die Seife und die Fettsäure in einem Verhältnis von 2:1 bis 10.000:1 vorliegen;
(c) 1 Teil bis 50 Teile eines schäumenden, synthetischen Tensids; und
(d) 10 Teile bis 50 Teile Wasser;

wobei das Wasser und das Lipid sich vorwiegend innerhalb Zwischenräumen des offenmaschigen, kristallinen Netzwerks befinden; wobei die Stückformzusammensetzung einen Lipid-Depositions-Wert von 3 bis 1.000 aufweist, und wobei die Stückform 0,1 bis 50 Teile weiterer Bestandteile enthält, gewählt aus

0,5 bis 5 Teilen Kaliumseife;
0,5 bis 5 Teilen alkoxylierten Ammonium- und/oder Alkylammonium- und/oder alkoxylierten aliphatischen Aminen und/oder polyethoxylierter Aminseife;
0,5 bis 15 Teilen Calciumseife;
0,5 bis 50 Teilen feinste wasserunlösliche Materialien, gewählt aus Calciumcarbonat und Talkum;
0,5 bis 25 Teilen Aluminosilicat-Ton und/oder andere Tone; wobei die Aluminosilicate und Tone gewählt sind aus Zeolithen, Kaolin, Kaolinit, Bentonit, Halloysit, calcinierten Tonen, Montmorilloniten, Illit und Attapulgit;
0,5 bis 25 Teilen Dextrin, Mais- und Getreidestärke;
0,1 bis 15 Teilen Salz und Salzhydrate; und Mischungen hiervon; und wobei das Salz und das Salzhydrat ein Kation aufweisen, gewählt aus Natrium, Kalium, Magnesium, Calcium, Aluminium, Lithium, Ammonium, alkoxyliertem Ammonium, Alkylammonium, alkoxylierten aliphatischen Aminen, polyethoxylierten Aminen; und wobei das Salz und/oder Salzhydrat ein Anion aufweisen, gewählt aus Chlorid, Sulfat, Isethionat, Bromid, Metasilicat, Orthophosphat, Pyrophosphat, Polyphosphat, Metaborat, Tetraborat, Carbonat, Bicarbonat, Hydrogenphosphat, Methylsulfat und Mono- und Polycarboxylat mit 1 bis 6 Kohlenstoffatomen oder weniger;
0,1 bis 1 Teilen Weißfärbehilfe;
0,1 bis 2 Teilen eines Duftstoffs oder Parfüms;
0,1 bis 5 Teilen eine Komplexierungsmittels, Konservierungsmittels oder antifungalen/anti mikrobiellen/bakteriellen Mittels.

**11.** Schäumende Hautreinigungszusammensetzung in Stückform nach mindestens einem der vorangehenden Ansprüche, umfassend, bezogen auf Gewichtsteile der Stückformzusammensetzung:

(a) 5 Teile bis 40 Teile eines Lipid-Hautfeuchthaltemittels, gewählt aus der Gruppe organischer Lipide, welche hydrophob sind, wie dadurch definiert, dass sie einen Vaughan-Solubilitäts-Parameter (VSP) von 5 bis weniger als 10 aufweisen; und Mischungen hiervon;
(b) 15 bis 40 Teile einer starren, semikontinuierlichen, ineinandergreifenden, offenmaschigen, kristallinen Netzwerkstruktur, bestehend im wesentlichen aus Mischungen aus Mono- und Dicarboxylfettäsureseifenmaterial, gewählt aus Mischungen aus Mono- und Dicarboxylfettsäureseife und Mono- und Dicarboxylfettsäure; wobei das Fettsäureseifenmaterial mindestens 75% gesättigte Alkylketten aufweist; die Alkylketten gewählt sind aus Ketten mit 8 bis 22 Kohlenstoffatomen, und Mischungen hiervon; und wobei die Seife und die Fettsäure ein Verhältnis von 2:1 bis 10.000:1 aufweisen;
(c) 1 Teil bis 50 Teile eines schäumenden, synthetischen Tensids; und
(d) 10 Teile bis 50 Teile Wasser;

wobei das Wasser und das Lipid sich vorwiegend innerhalb Zwischenräumen des offenmaschigen, kristallinen Netzwerks befinden; wobei die Stückformzusammensetzung einen Lipid-Depositions-Wert von mindestens 3 bis

1.000 aufweist.

**12.** Schäumende Hautreinigungszusammensetzung in Stückform nach Anspruch 1, wobei das Lipid einen Scherindex bei 35°C im Bereich von 0,1 bis 0,5 und eine Konsistenz k bei 35°C im Bereich von 10 bis 3.000 poise aufweist; und wobei das Lipid vorzugsweise einen bei 1 Hz und 35°C gemessenen Elastizitätsmodul (G') im Bereich von 25 bis 100.000 dynes/$cm^2$ und einen bei 1 Hz und 35°C gemessenen Viskositätsmodul (G") im Bereich von 25 bis 500.000 dynes/$cm^2$ aufweist; und wobei der Elastiziätsmodul (G') weiter vorzugsweise im Bereich von 5.000 bis 50.000 dynes/$cm^2$ und der Viskositätsmodul (G") im Bereich von 5.000 bis 100.000 dynes/$cm^2$ liegt.

**13.** Schäumende Hautreinigungszusammensetzung in Stückform nach Anspruch 2, wobei das Lipid 12 bis 25 Gewichtsteile der Stückformzusammensetzung ausmacht; und wobei das Lipid einen k-Wert von 50 bis 2.000 poise aufweist; und wobei mindestens 70% des Lipids zusammengesetzt sind aus Lipiden, gewählt aus Petrolatum, Mineralöl, mikrokristallinem Wachs, Paraffin, Polyethylen, Polybuten, Polydecen, Dimethiconen, Alkylsiloxan, Cyclomethiconen, Lanolin, Lanolinöl, Lanolinwachs, wobei der Rest gewählt ist aus: Isopropylpalmitat, Cetylriconoleat, Octylisononanoat, Octylpalmitat, Isocetylstearat, hydroxyliertem Milchglycerid; und Mischungen hievon; und wobei das Wasser eine wäßrige Phase bildet, wobei 20 bis 95% der wäßrigen Phase Wasser sind, und wobei das Verhältnis von Wasser zu den Lipidteilen vorzugsweise größer als 1:1 ist; und wobei das Wasser 15 bis 35 Teile ausmacht; und wobei die wäßrige Phase und das Lipid eine Lipid-in-Wasser-Emulsion bilden, welche in dem ineinandergreifenden, offenmaschigen, kristallinen Netzwerk eingeschlossen ist; und wobei die Stückform eine instabile Lipid-in-Wasser-Emulsion bildet, wenn sie einem Lipid-Stabilitäts-Zentrifugen-Test unterzogen wird.

**Revendications**

**1.** Composition de pain moussant pour le nettoyage de la peau comprenant *en parties en poids de la composition de pain* :

(a) 5 parties à 40 parties d'un agent d'hydratation cutanée lipidique, choisi dans le groupe des lipides organiques, qui sont hydrophobes au sens qu'ils ont un Paramètre de Solubilité Vaughan (VSP) combiné de 5 à moins de 10 ; et de leurs mélanges ;
(b) 15 parties à 40 parties d'une structure en réseau cristallin à mailles ouvertes enchevêtrées, semi-continue, rigide, constituée essentiellement d'un matériau de savon d'acide gras qui est un mélange de savon d'acide gras et d'acide gras, ledit matériau de savon d'acide gras ayant au moins 75 % de chaînes alkyle saturées ; lesdites chaînes alkyle étant choisies parmi les chaînes de 8 à 22 atomes de carbone, et leurs mélanges ; ledit savon et ledit acide gras étant présents dans un rapport de 2:1 à 10 000:1 ;
(c) 1 partie à 50 parties d'un tensioactif synthétique moussant ; et
(d) 10 parties à 50 parties d'eau ;

dans laquelle ladite eau et ledit lipide se trouvent essentiellement dans les interstices dudit réseau cristallin à mailles ouvertes ; dans laquelle ladite composition de pain a une Valeur de Dépôt de Lipide de 3 à 1 000.

**2.** Composition de pain moussant pour le nettoyage de la peau de la revendication 1 dans laquelle au moins 80 % dudit matériau de savon d'acides gras a la structure suivante :

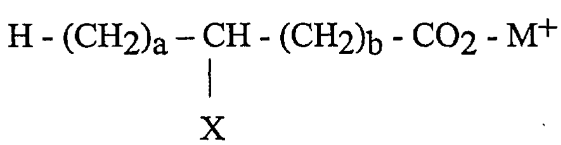

dans laquelle:

a + b = 8 à 20
chaque a, b = 0 à 20
X = H, OR, O-CO-R, R, ou des mélanges de ceux-ci
R = alkyle en $C_1$-$C_3$, H, ou des mélanges de ceux-ci
M = Na, (1/2Mg), ou des mélanges de ceux-ci ; et,

dans laquelle ledit matériau de savon d'acides gras a au moins 85 % de chaînes alkyle saturées, environ 80 % à 100 % desdites chaînes alkyle saturées étant choisies dans le groupe constitué des chaînes de 12 à 18 atomes de carbone ; et dans laquelle ledit lipide est présent à raison de 10 à 40 parties *en poids de la composition de pain* ; ledit lipide ayant une valeur de consistance k en viscosité de 5 poises à 5 000 poises à 35°C ; et ledit lipide ayant un indice de cisaillement à 35°C dans la gamme de 0,1 à 0,8.

**3.** Composition de pain moussant pour le nettoyage de la peau de la revendication 2 ; dans laquelle ledit tensioactif synthétique moussant est présent à raison de 5 à 40 parties *en poids de la composition de pain* ; et dans laquelle ledit tensioactif synthétique moussant est choisi parmi les tensioactifs anioniques; les tensioactifs non ioniques; les tensioactifs amphotères ; et leurs mélanges; et dans laquelle ledit tensioactif synthétique moussant a une valeur de tension superficielle d'équilibre à la concentration critique pour la formation de micelles (CMC) de 15 à 50 dynes par cm à 25°C ; et ladite eau est présente à raison de 15 à 45 parties ; et dans laquelle ledit lipide est présent à raison de 10 à 35 parties *en poids de la composition de pain*; et dans laquelle ledit lipide est choisi parmi les huiles et cires hydrocarbonées, les huiles de silicone, les graisses et huiles de di- et tri-glycérides, les esters d'acétoglycérides, les esters d'alkyle, les esters d'alcényle, les polyesters d'acides gras de polyols, la lanoline et ses dérivés, les esters de cire, les dérivés de cire d'abeilles, les cires végétales, les stérols et les phospholipides ; et dans laquelle ledit pain a une Valeur de Dépôt de Lipide de 5 à 500 ;

**et** dans laquelle

ladite huile et cire hydrocarbonée est **de préférence** choisie parmi la vaseline, l'huile minérale, les cires microcristallines, les polyalcènes, la paraffine, la cérasine, l'ozocérite, le polyéthylène et le perhydrosqualène ;
ladite huile de silicone est **de préférence** choisie parmi les diméthicones, les cyclométhicones, les alkylsiloxanes, les polyméthylsiloxanes et les méthylphénylpolysiloxanes ;
ledit di- et tri-glycéride est **de préférence** choisi parmi le glycéride de lait hydroxylé, l'huile de ricin, l'huile de soja, l'huile de soja modifiée avec un maléate, l'huile de carthame, l'huile de coton, l'huile de maïs, l'huile de noix, l'huile d'arachide, l'huile d'olive, l'huile de foie de morue, l'huile d'amande douce, l'huile d'avocat, l'huile de palme et l'huile de sésame ;
ledit ester d'alkyle est **de préférence** choisi parmi les esters isopropyliques d'acides gras et les esters alkyliques de l'acide ricinoléique, le palmitate d'isopropyle, le myristate d'isopropyle, le ricinoléate de cétyle et le ricinoléate de stéaryle; le laurate d'hexyle, le laurate d'isohexyle, le myristate de myristyle, le palmitate d'isohexyle, l'oléate de décyle, l'oléate d'isodécyle, le stéarate d'hexadécyle, le stéarate de décyle, l'isostéarate d'isopropyle, l'adipate de diisopropyle, l'adipate de diisohexyle, l'adipate de dihexyldécyle, le sébaçate de diisopropyle, l'isononanoate d'acyle, le lactate de lauryle, le lactate de myristyle, le myristate d'oléyle, le stéarate d'oléyle et l'oléate d'oléyle, et le lactate de cétyle;
ladite substance à base de lanoline est **de préférence** choisie parmi l'huile de lanoline, la cire de lanoline, l'alcool de lanoline, l'acide gras de lanoline, le lanolate d'isopropyle, la lanoline acétylée, les alcools de lanoline acétylée, le linoléate d'alcool de lanoline, le ricinoléate d'alcool de lanoline ;
ledit ester de cire est **de préférence** choisi parmi la cire d'abeilles et les dérivés de cire d'abeilles, le blanc de baleine, le myristate de myristyle, le stéarate de stéaryle ;
ladite cire végétale est **de préférence** choisie parmi les cires de carnauba et de candélilla ;
ledit stérol est **de préférence** choisi parmi le cholestérol, les esters d'acides gras de cholestérol et leurs homologues ;
ledit phospholipide est **de préférence** choisi parmi la lécithine et ses dérivés, les sphingolipides, les céramides, les glycosphingolipides ; et leurs homologues ; et les mélanges de ceux-ci ;

et dans laquelle ledit lipide est présent **de préférence** à raison de 10 parties à 30 parties, en poids de la composition de pain ; et dans laquelle ledit pain a une Valeur de Dépôt de Lipide dans la gamme de 10 à 500 ; et dans laquelle **de préférence** au moins 70 % de ladite phase lipidique se compose de lipides choisis parmi la vaseline, l'huile minérale, les cires microcristallines, les polyalcènes, la paraffine, la cérasine, l'ozocérite, le polyéthylène et le perhydrosqualène ; les diméthicones, les cyclométhicones, les alkylsiloxanes, les polyméthylsiloxanes et les méthylphénylpolysiloxanes ; le glycéride de lait hydroxylé, l'huile de ricin, l'huile de soja, l'huile de soja modifiée avec un maléate, l'huile de carthame, l'huile de coton, l'huile de maïs, l'huile de noix, l'huile d'arachide, l'huile d'olive, l'huile de foie de morue, l'huile d'amande douce, l'huile d'avocat, l'huile de palme et l'huile de sésame ; la lanoline, l'huile de lanoline, la cire de lanoline, l'alcool de lanoline, l'acide gras de lanoline, le lanolate d'isopropyle, la lanoline acétylée, les alcools de lanoline acétylée, le linoléate d'alcool de lanoline, le ricinoléate d'alcool de lanoline ; et leurs mélanges.

**4.** Composition de pain moussant pour le nettoyage de la peau de la revendication 2 dans laquelle ledit tensioactif synthétique anionique est présent à raison de 3 à 35 parties et dans laquelle ledit tensioactif synthétique anionique moussant est choisi parmi les iséthionates d'acyle, les sarcosinates d'acyle, les éthers de glycéryle d'alkylsulfonates, les méthylacyltaurates, les paraffinesulfonates, les (alkyl linéaire)benzènesulfonates, les N-acylglutamates, les alkylsulfosuccinates, les esters d'alpha-sulfoacide gras, les éthercarboxylates d'alkyle, les esters d'alkylphosphate, les esters d'alkylphosphate éthoxylés, les oxydes d'alkylamine, les alpha-oléfinesulfates, les alkyléthersulfates (avec 1 à 12 groupes éthoxy), et leurs mélanges, où lesdits tensioactifs contiennent des chaînes alkyles en $C_8$ à $C_{22}$ et où le contre-ion est choisi parmi Na, K, $NH_4$, $N(CH_2CH_2OH)_3$.

**5.** Composition de pain moussant pour le nettoyage de la peau de la revendication 4 dans laquelle le matériau d'acide gras est présent à raison de 15 parties à 35 parties ; dans laquelle au moins 25 % du matériau de savon d'acides gras ont des chaînes alkyle de 12 à 14 atomes de carbone, où au moins 95 % desdites chaînes alkyle sont saturées ; et dans laquelle ledit savon représente au moins quatre fois ledit acide gras ; et dans laquelle ledit savon d'acides gras est choisi parmi au moins 75 % de savon de sodium ; et dans laquelle ledit lipide est présent à raison de 12 parties à 24 parties en poids du pain ; dans laquelle ledit pain a une Valeur de Dépôt de Lipide de 25 à 500 ; et dans laquelle ladite eau est présente à raison de 15 à 35 parties, en poids ;
et dans laquelle ledit tensioactif anionique est **de préférence** présent à raison de 8 à 30 parties, choisi parmi les acyliséthionates, les acylsarcosinates, les alkylsulfosuccinates, les éther de glycéryle d'alkylsulfonates, les méthylacyltaurates, les alkyléthersulfates, les alkylsulfates, les esters d'alkylphosphate, et leurs mélanges, où lesdits tensioactifs contiennent des chaînes alkyles en $C_8$ à $C_{18}$; et dans laquelle le contre-ion est Na ; et dans laquelle ledit tensioactif synthétique amphotère est **de préférence** présent à raison de 1 à 10 parties, choisi parmi les alkylampho-mono- et di-acétates, les alkylbétaïnes, les alkylsultaïnes, les alkylamidopropylbétaïnes, les alkylamidopropylhydroxysultaïnes, et leurs mélanges, où ledit tensioactif amphotère contient des chaînes alkyle en $C_{12}$ à $C_{22}$ ; et dans laquelle ledit tensioactif synthétique moussant a une valeur de tension superficielle d'équilibre à la concentration critique de formation de micelles (CMC) de 15 à 45 dynes par cm; et dans laquelle ledit tensioactif synthétique moussant est présent **de préférence** à raison de 10 à 35 parties, en poids; et ce dernier est **de préférence** choisi parmi un tensioactif synthétique anionique et/ou amphotère où ledit tensioactif anionique est plus **préférentiellement** choisi parmi le lauryliséthionate et le coprah-iséthionate de sodium, les laurylsarcosinates et coprah-sarcosinates de sodium, les alkyl(en $C_{12}$-$C_{16}$)sulfosuccinates de sodium, les (alkyl en $C_{12}$-$C_{16}$)glycéryléthersulfonates, les lauryltaurates et coprah-taurates de sodium ; et où ledit tensioactif amphotère est **de préférence** choisi parmi les laurylbétaïnes et coprah-bétaïnes, les laurylhydroxysultaïnes et coprahhydroxysultaïnes, et leurs mélanges; et dans laquelle le rapport dudit tensioactif anionique audit tensioactif amphotère est **de préférence** de 1:1 à 30:1.

**6.** Composition de pain moussant pour le nettoyage de la peau de la revendication 5, dans laquelle ledit tensioactif synthétique comprend 3 à 17 parties d'un tensioactif synthétique moussant non ionique choisi parmi les alkylglucosamides, les glucose-esters d'alkyle, les polyoxyéthylèneamides, les alcanamides gras, les oxydes d'alkylamine, les alkylpolyglucosides, les alkylphénols de polyoxyéthylène, les esters de polyoxyéthylène d'acides gras, les copolymères séquencés OE/OP tels que les polyoxamines et les poloxamères, les esters d'alcools et esters de sorbitane, et leurs mélanges.

**7.** Composition de pain moussant pour le nettoyage de la peau selon l'une quelconque des revendications précédentes comprenant *en parties en poids de la composition de pain*:

(a) 5 parties à 40 parties d'un agent d'hydratation cutanée lipidique, choisi dans le groupe des lipides organiques, qui sont hydrophobes au sens qu'ils ont un Paramètre de Solubilité Vaughan (VSP) de 5 à moins de 10 ; et de leurs mélanges ;
(b) 15 parties à 40 parties d'une structure en réseau cristallin à mailles ouvertes enchevêtrées, semi-continue, rigide, constituée essentiellement d'un matériau de savon d'acide gras qui est un mélange de savon d'acide gras et d'acide gras, ledit matériau de savon d'acide gras ayant au moins 75 % de chaînes alkyle saturées ; lesdites chaînes alkyle étant choisies parmi les chaînes de 8 à 22 atomes de carbone, et leurs mélanges ; ledit savon et ledit acide gras étant présents dans un rapport de 2:1 à 10 000:1 ;
(c) 1 partie à 50 parties d'un tensioactif synthétique moussant ; et
(d) 10 parties à 50 parties d'eau;
(e) 0,5 à 6 parties d'un tensioactif cationique; et

dans laquelle ladite eau et ledit lipide se trouvent essentiellement dans les interstices dudit réseau cristallin à mailles ouvertes; dans laquelle ladite composition de pain a une Valeur de Dépôt de Lipide de 3 à 1 000; et dans

laquelle ledit tensioactif cationique améliore la Valeur de Dépôt de Lipide; et dans laquelle ledit tensioactif synthétique cationique est **de préférence** choisi parmi le chlorure et le méthylsulfate d'alkyltrimonium, le chlorure et le méthylsulfate d'alkylalconium, et le chlorure et le méthylsulfate de dialkyldimonium, et leurs mélanges, où lesdits tensioactifs cationiques contiennent **de préférence** 12 à 14 atomes de carbone par chaîne alkyle ; et dans laquelle ledit tensioactif cationique est plus **préférentiellement** choisi parmi le chlorure de stéaralconium, le chlorure de stéaryltrimonium, le chlorure de distéaryl-dimonium, et leurs mélanges.

**8.** Composition de pain moussant pour le nettoyage de la peau selon l'une quelconque des revendications précédentes comprenant *en parties en poids de la composition de pain* :

(a) 5 parties à 40 parties d'un agent d'hydratation cutanée lipidique, choisi dans le groupe des lipides organiques, qui sont hydrophobes au sens qu'ils ont un Paramètre de Solubilité Vaughan (VSP) de 5 à moins de 10 ; et de leurs mélanges ;
(b) 15 parties à 40 parties d'une structure en réseau cristallin à mailles ouvertes enchevêtrées, semi-continue, rigide, constituée essentiellement d'un matériau de savon d'acide gras qui est un mélange de savon d'acide gras et d'acide gras, ledit matériau de savon d'acide gras ayant au moins 75 % de chaînes alkyle saturées; lesdites chaînes alkyle étant choisies parmi les chaînes de 8 à 22 atomes de carbone, et leurs mélanges; ledit savon et ledit acide gras étant présents dans un rapport de 2:1 à 10 000:1;
(c) 1 partie à 50 parties d'un tensioactif synthétique moussant ; et
(d) 10 parties à 50 parties d'eau ;
(e) 0,5 à 35 parties d'une substance organique soluble dans l'eau choisie parmi un polyol de structure:

$$R^1 - O(CH_2 - CR^2HO)_nH$$

dans laquelle $R^1$ = H, alkyle en $C_1$-$C_4$; $R^2$ = H, $CH_3$ et n = 1-200; les alcanediols en $C_2$-$C_{10}$; la guanidine; l'acide glycolique et les sels glycolate (par exemple d'ammonium et d'alkylammonium quaternaire); l'acide lactique et les sels lactate (par exemple d'ammonium et d'alkylammonium quaternaire); les alcools polyhydroxylés tels que le sorbitol, le glycérol, l'hexanetriol, le propylèneglycol, l'hexylèneglycol et analogues; le polyéthylèneglycol; les sucres et amidons; les dérivés de sucre et d'amidon (par exemple le glucose alcoxylé); le panthénol (comprenant les formes D, L, et D,L); l'acide pyrrolidonecarboxylique ; l'acide hyaluronique; la lactamidemonoéthanolamine; l'acétamidemonoéthanolamine; l'urée; et les éthanolamines de structure générale $(HOCH_2CH_2)_xNH_y$ où x = 1-3 ; y = 0-2, et x+y = 3, et leurs mélanges ; dans laquelle ledit pain contient une substance organique non volatile ayant une solubilité d'au moins 50 % dans l'eau ; et dans laquelle ladite substance organique soluble dans l'eau est soluble à au moins 50 % dans l'eau ; et dans laquelle ladite eau et ledit lipide se trouvent essentiellement dans les interstices dudit réseau cristallin à mailles ouvertes ; dans laquelle ladite composition de pain a une Valeur de Dépôt de Lipide de 3 à 1 000.

**9.** Composition de pain moussant pour le nettoyage de la peau de la revendication 8 dans laquelle ladite partie substance organique soluble dans l'eau est présente à raison de 3 à 20 parties, en poids dudit pain ; et dans laquelle ladite substance organique est choisie parmi la glycérine, le polyoxypropylène (1) glycérol et le polyoxypropylène (3) glycérol, le sorbitol, le butylèneglycol, le propylèneglycol, le saccharose, et l'urée et la triéthanolamine ; et leurs mélanges ; et dans laquelle ledit lipide est présent à raison de 15 parties à 25 parties, en poids du pain ; et dans laquelle ledit tensioactif synthétique est présent à raison de 15 à 30 parties en poids du pain ; ledit savon est présent à raison de 15 parties à 25 parties et ledit savon consiste essentiellement en acides carboxyliques en $C_{12}$-$C_{18}$, au moins 50 % étant en $C_{12}$ ou $C_{14}$; et dans laquelle au moins 75 % dudit lipide en poids dudit lipide est choisi parmi la vaseline, la cire microcristalline, l'huile minérale ou le polydécène et leurs mélanges ; et dans laquelle 0 à 25 % dudit lipide en poids dudit lipide est choisi parmi la diméthicone ou un alkylpolysiloxane et leurs mélanges ; et dans laquelle ladite eau est **de préférence** présente à raison de 15 parties à 35 parties ; et dans laquelle ledit lipide est **de préférence** présent à raison de 15 à 25 parties, en poids de la composition, au moins 75 % dudit lipide étant choisi parmi la vaseline, la cire microcristalline, l'huile minérale ou le polydécène ; et dans laquelle ledit lipide a un module élastique (G') mesuré à 1 Hz et 35°C dans la gamme de 1 000 à 80 000 dynes/$cm^2$ et a un module visqueux (G") mesuré à 1 Hz et 35°C dans la gamme de 500 à 300 000 dynes/$cm^2$ ; et dans laquelle ledit pain contient 0,1 partie à 10 parties d'un adjuvant de sensation sur la peau polymère non ionique, cationique, ou modifié hydrophobiquement, choisi parmi les polysaccharides cationiques de la catégorie de la gomme de guar cationique avec des poids moléculaires de 1 000 à 3 000 000, les homopolymères cationiques et non ioniques dérivés de l'acide acrylique et/ou méthacrylique, les résines de cellulose cationiques et non ioniques; les copolymères cationiques de chlorure de diméthyldialkylammonium et d'acide

acrylique; les homopolymères cationiques de chlorure de diméthyldialkylammonium ; les polyalkylène et éthoxypolyalkylène imines cationiques ; et leurs mélanges, et dans laquelle ledit adjuvant de sensation sur la peau polymère non ionique ou cationique est **de préférence** présent à raison de 0,25 parties à 3 parties, en poids, et est **de préférence** choisi parmi l'hydroxypropylguar, le chlorure de guarhydroxypropyltrimonium, les polyquaternaires 3, 5, 6, 7, 10, 11 et 24 et leurs mélanges.

**10.** Composition de pain moussant pour le nettoyage de la peau selon l'une quelconque des revendications précédentes comprenant *en parties en poids de la composition de pain*:

(a) 5 parties à 40 parties d'un agent d'hydratation cutanée lipidique, choisi dans le groupe des lipides organiques, qui sont hydrophobes au sens qu'ils ont un Paramètre de Solubilité Vaughan (VSP) de 5 à moins de 10 ; et de leurs mélanges;
(b) 15 parties à 40 parties d'une structure en réseau cristallin à mailles ouvertes enchevêtrées, semi-continue, rigide, constituée essentiellement d'un matériau de savon d'acide gras qui est un mélange de savon d'acide gras et d'un acide gras, ledit matériau de savon d'acide gras ayant au moins 75 % de chaînes alkyle saturées; lesdites chaînes alkyle étant choisies parmi les chaînes de 8 à 22 atomes de carbone, et leurs mélanges ; ledit savon et ledit acide gras étant présents dans un rapport de 2:1 à 10 000:1 ;
(c) 1 partie à 50 parties d'un tensioactif synthétique moussant ; et
(d) 10 parties à 50 parties d'eau;

dans laquelle ladite eau et ledit lipide se trouvent essentiellement dans les interstices dudit réseau cristallin à mailles ouvertes ; dans laquelle ladite composition de pain a une Valeur de Dépôt de Lipide de 3 à 1 000, et dans laquelle ledit pain contient 0,1 partie à 50 parties d'autres ingrédients choisis parmi:

0,5 à 5 parties de savon de potassium ;
0,5 à 5 parties de savon d'ammonium alcoxylé et/ou d'alkylammonium et/ou d'amines aliphatiques alcoxylées et/ou d'amines polyéthoxylées;
0,5 à 15 parties de savon de calcium ;
0,5 à 50 parties de substances insolubles dans l'eau impalpables choisies parmi le carbonate de calcium et le talc;
0,5 à 25 parties d'argile d'aluminosilicates et/ou d'autres argiles; où lesdits aluminosilicates et argiles sont choisis parmi les zéolithes, le kaolin, la kaolinite, la bentonite, l'halloysite, les argiles calcinées, la montmorillonite, l'illite et l'attapulgite ;
0,5 à 25 parties de dextrine, d'amidon de maïs et de blé ;
0,1 à 15 parties de sel et d'hydrates de sel ; et de leurs mélanges; et où lesdits sel et hydrate de sel ont un cation choisi parmi les sodium, potassium, magnésium, calcium, aluminium, lithium, ammonium, ammonium alcoxylé, alkylammonium, amines aliphatiques alcoxylées, amines polyéthoxylées ; et où lesdits sel et/ou hydrate de sel ont un anion choisi parmi les chlorure, sulfate, iséthionate, bromure, métasilicate, orthophosphate, pyrophosphate, polyphosphate, métaborate, tétraborate, carbonate, bicarbonate, hydrogénophosphate, méthylsulfate, et mono- et
polycarboxylate de 1 à 6 atomes de carbone ou moins ;
0,1 à 1 partie d'un adjuvant de blanchiment;
0,1 à 2 parties d'un arôme ou d'un parfum;
0,1 à 5 parties d'un agent chélatant, d'un conservateur ou d'un agent antifongique/antimicrobien/antibactérien.

**11.** Composition de pain moussant pour le nettoyage de la peau selon l'une quelconque des revendications précédentes comprenant *en parties en poids de la composition de pain*:

(a) 5 parties à 40 parties d'un agent d'hydratation cutanée lipidique, choisi dans le groupe des lipides organiques, qui sont hydrophobes au sens qu'ils ont un Paramètre de Solubilité Vaughan (VSP) de 5 à moins de 10; et de leurs mélanges ;
(b) 15 parties à 40 parties d'une structure en réseau cristallin à mailles ouvertes enchevêtrées, semi-continue, rigide, constituée essentiellement de mélanges de matériau de savon d'acides gras mono- et di-carboxyliques choisis parmi les mélanges de savon d'acides gras mono- et di-carboxyliques et d'un acide gras mono- et di-carboxylique; ledit matériau de savon d'acides gras ayant au moins 75 % de chaînes alkyle saturées; lesdites chaînes alkyle étant choisies parmi les chaînes de 8 à 22 atomes de carbone, et leurs mélanges; ledit savon et ledit acide gras étant présents dans un rapport de 2:1 à 10 000:1;
(c) 1 partie à 50 parties d'un tensioactif synthétique moussant ; et

(d) 10 parties à 50 parties d'eau ;

dans laquelle ladite eau et ledit lipide se trouvent essentiellement dans les interstices dudit réseau cristallin à mailles ouvertes ; dans laquelle ladite composition de pain a une Valeur de Dépôt de Lipide d'au moins 3 à 1 000.

**12.** Composition de pain moussant pour le nettoyage de la peau de la revendication 1, dans laquelle ledit lipide a un indice de cisaillement à 35°C dans la gamme de 0,1 à 0,5 et une consistance k à 35°C dans la gamme de 10 à 3 000 poises ; et dans laquelle ledit lipide **de préférence** a un module élastique (G') mesuré à 1 Hz et 35°C dans la gamme de 25 à 100 000 dynes/$cm^2$ et a un module visqueux (G") mesuré à 1 Hz et 35°C dans la gamme de 25 à 500 000 dynes/$cm^2$ ; et dans laquelle ledit module élastique (G') est plus **préférentiellement** dans la gamme de 5 000 à 50 000 dynes/$cm^2$ et a un module visqueux (G") dans la gamme de 5 000 à 100 000 dynes/$cm^2$.

**13.** Composition de pain moussant pour le nettoyage de la peau de la revendication 2, dans laquelle ledit lipide est présent à raison de 12 parties à 25 parties, en poids de la composition de pain ; et dans laquelle ledit lipide a une valeur k de 50 à 2 000 poises ; et dans laquelle au moins 70 % dudit lipide se compose de lipides choisis parmi la vaseline, l'huile minérale, la cire microcristalline, la paraffine, le polyéthylène, le polybutène, le polydécène, les diméthicones, un alkylsiloxane, les cyclométhicones, la lanoline, l'huile de lanoline, la cire de lanoline ; le reste étant choisi parmi le palmitate d'isopropyle, le ricinoléate de cétyle, l'isononanoate d'octyle, le palmitate d'octyle, le stéarate d'isocétyle, le glycéride de lait hydroxylé ; et leurs mélanges ; et dans laquelle ladite eau forme une phase aqueuse où 20 à 95 % de ladite phase aqueuse est de l'eau, et dans laquelle le rapport desdites parties d'eau auxdites parties de lipide est de préférence supérieur à 1:1; et dans laquelle ladite eau est présente à raison de 15 à 35 parties ; et dans laquelle ladite phase aqueuse et ledit lipide forment une émulsion lipide-dans-eau emprisonnée dans ledit réseau cristallin à mailles ouvertes enchevêtrées ; et dans laquelle ledit pain produit une émulsion lipide-dans-eau instable lorsqu'il est soumis à un Test Centrifuge de Stabilité du Lipide.

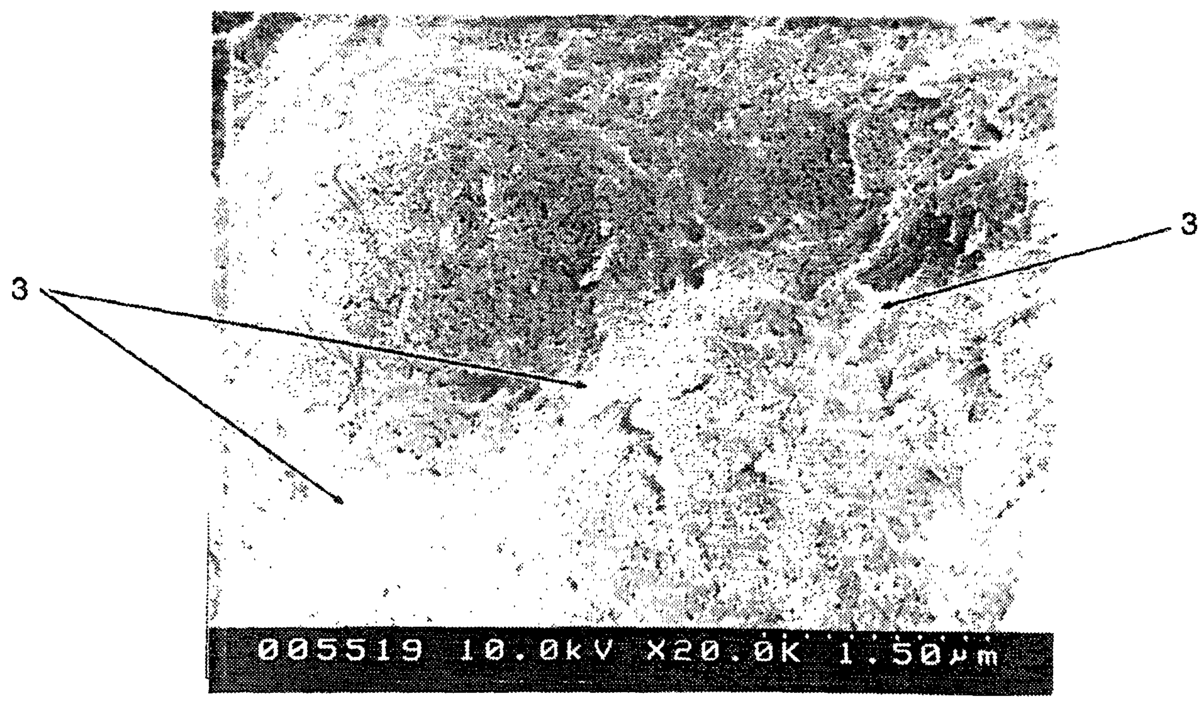


Fig. 1

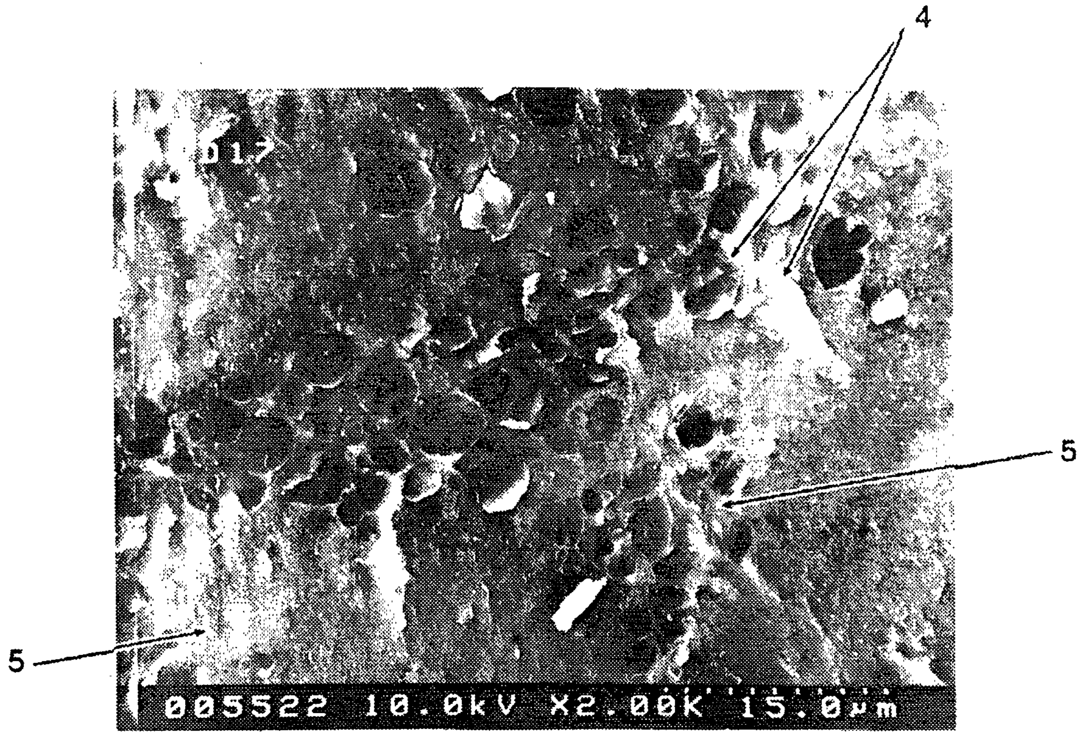


Fig. 2

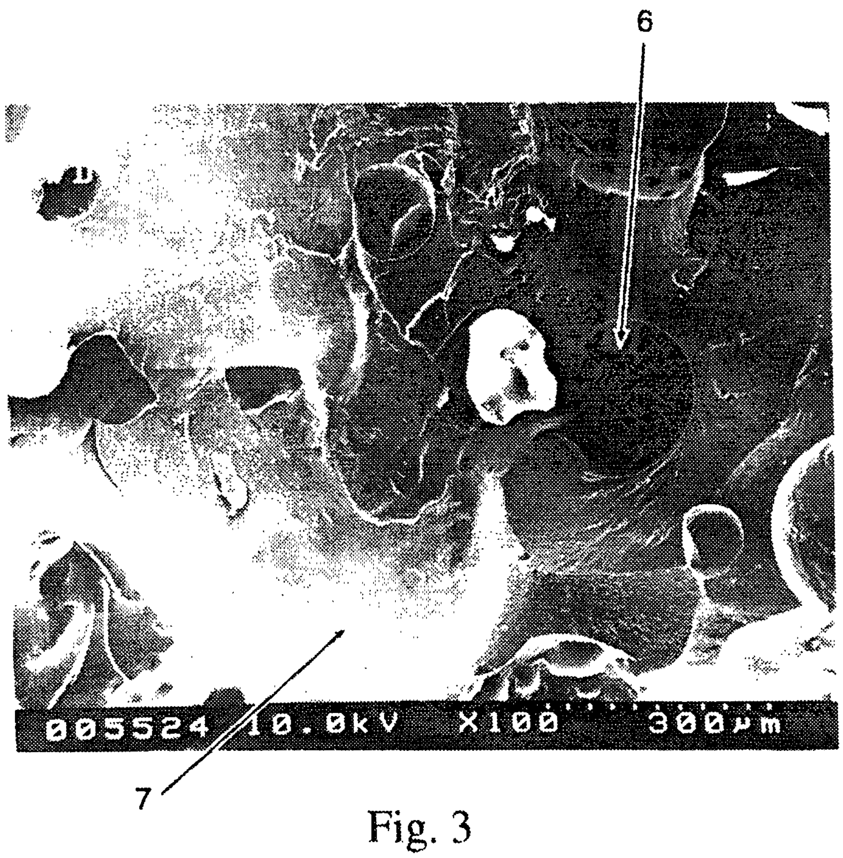


Fig. 3

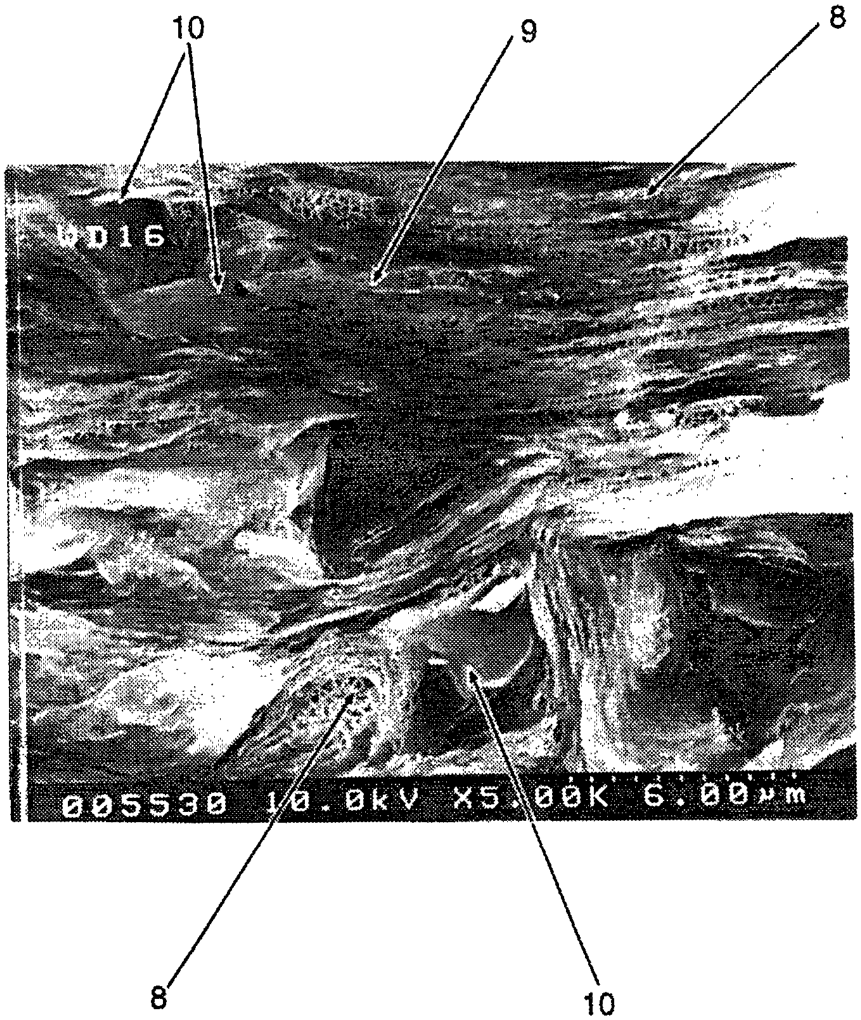


Fig. 4

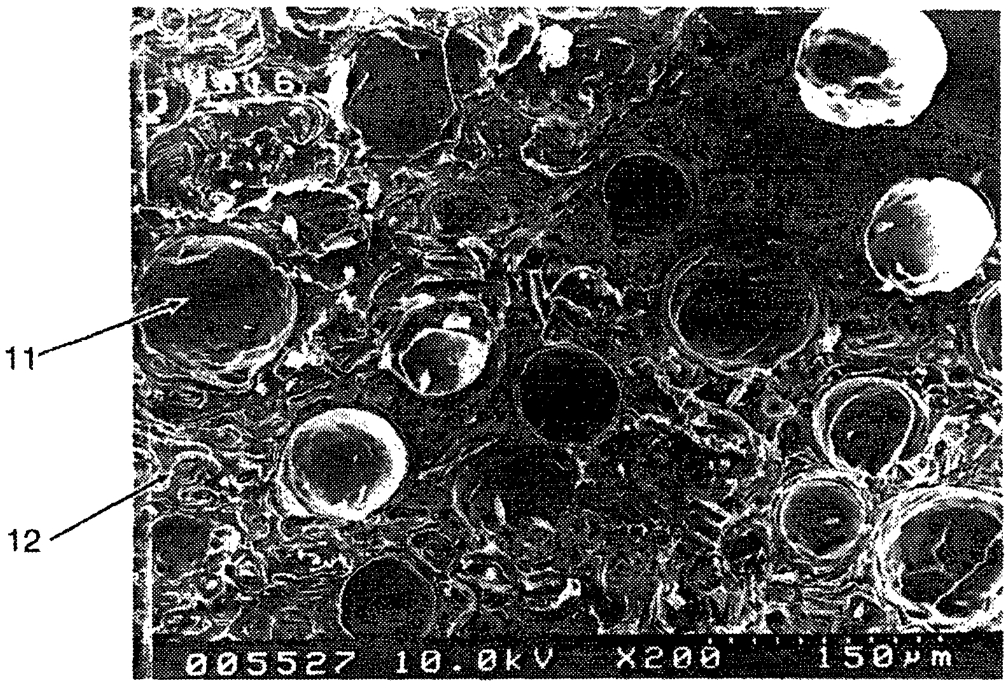


Fig. 5